# EUROPÄISCHE PATENTSCHRIFT

(11) **EP 3 131 994 B1**
(45) Veröffentlichungstag und Bekanntmachung des Hinweises auf die Patenterteilung: **09.06.2021**
(21) Anmeldenummer: 15718192.6
(22) Anmeldetag: 16.04.2015
(51) Int. Cl.: C09D 183/10, C07C 279/00, C08G 77/42, C09J 183/10

(54) **SCHNELL HÄRTENDE MIGRATIONSFREIE ZUSAMMENSETZUNG AUF BASIS VON SILANGRUPPEN-HALTIGEN ORGANISCHEN POLYMEREN**
FAST CURING MIGRATION-FREE COMPOSITION BASED ON ORGANIC POLYMERS CONTAINING SILANE GROUPS
COMPOSITION SANS MIGRATION À DURCISSEMENT RAPIDE À BASE DE POLYMÈRES ORGANIQUES CONTENANT DES GROUPES SILANES

(30) Priorität: 16.04.2014 EP 14164920
(43) Veröffentlichungstag der Anmeldung: 22.02.2017
(73) Patentinhaber: Sika Technology AG, 6340 Baar (CH)
(72) Erfinder: BURCKHARDT, Urs, CH-8049 Zürich (CH); CANNAS, Rita, CH-8600 Dübendorf (CH)
(74) Vertreter: Sika Patent Attorneys
(86) Internationale Anmeldenummer: PCT/EP2015/058332
(87) Internationale Veröffentlichungsnummer: WO 2015/158863

(56) Entgegenhaltungen:
- EP-A1- 1 985 666
- EP-A1- 2 100 923
- EP-A1- 2 388 297
- WO-A1-2010/043353
- WO-A2-2009/118307
- FR-A1- 2 925 496

## Beschreibung

### Technisches Gebiet

Die Erfindung betrifft bei Raumtemperatur härtbare Zusammensetzungen auf Basis von Silangruppen-haltigen organischen Polymeren, welche insbesondere geeignet sind als Klebstoff, Dichtstoff oder Beschichtung.

### Stand der Technik

Bei Raumtemperatur härtbare Zusammensetzungen auf Basis von Silangruppen-haltigen organischen Polymeren, welche auch als "silanfunktionelle Polymere", "silanmodifizierte Polymere" (SMP) oder "silanterminierte Polymere" (STP) bezeichnet werden, spielen eine bedeutende Rolle in vielen technischen Anwendungen, beispielsweise als Klebstoffe, Dichtstoffe oder Beschichtungen. Ihre Aushärtung erfolgt über Vernetzungsreaktionen der Silangruppen, welche unter dem Einfluss von Feuchtigkeit hydrolysieren, als Silanolgruppen untereinander kondensieren und dabei Siloxanbindungen bilden. Zur Beschleunigung der Aushärtung werden häufig Katalysatoren eingesetzt. Sehr oft handelt es sich dabei um toxikologisch bedenkliche Stoffe, welche eine potentielle Gefährdung von Verarbeiter und Umwelt darstellen, insbesondere auch nach der Aushärtung, wenn die Katalysatoren oder ihre Abbauprodukte durch Ausgasen, Migration oder Auswaschen freigesetzt werden.

Als Vernetzungskatalysatoren werden klassischerweise Organozinn-Verbindungen, insbesondere Dialkylzinn(IV)-carboxylate, eingesetzt. Diese zeichnen sich durch eine gute Aktivität in Bezug auf die Silanol-Kondensation aus und sind sehr hydrolysebeständig, allerdings sind sie gesundheitsschädlich und stark wassergefährdend. Oft werden sie in Kombination mit weiteren Katalysatoren verwendet, hauptsächlich basischen Verbindungen, insbesondere Amine, welche vor allem die vorgeschaltete Hydrolyse der Silangruppen beschleunigen.

Aufgrund einer stärkeren Gewichtung von EHS-Aspekten durch Berufsverbände und Verbraucher sowie schärferer staatlicher Regulierung werden seit einiger Zeit vermehrt Anstrengungen unternommen, die Organozinn-Verbindungen durch andere, weniger toxische Katalysatoren auszutauschen. So wurden etwa Organotitanate, -zirkonate und -aluminate als alternative Metall-Katalysatoren beschrieben. Diese haben aber zumeist eine geringere katalytische Aktivität in Bezug auf die Silanol-Kondensation als die Organozinn-Verbindungen und bewirken eine deutlich langsamere Vernetzung. Wegen ihrer mangelnden Hydrolysestabilität können sie beim Lagern der Zusammensetzung durch den Restfeuchtegehalt der Inhaltsstoffe einen Grossteil ihrer Aktivität verlieren, wodurch sich die Aushärtung stark verlangsamt oder ganz zum Erliegen kommt.

Eine weitere bekannte Alternative zu Organozinn-Verbindungen stellen stark basische Stickstoff-Verbindungen aus der Klasse der Amidine und Guanidine dar, welche in Kombination mit den erwähnten Metall-Katalysatoren oder auch allein eingesetzt werden können. Viele der gebräuchlichen Amidin- und Guanidin-Katalysatoren, wie insbesondere 1,8-Diazabicyclo[5.4.0]undec-7-en (DBU) und 1,1,3,3-Tetramethylguanidin (TMG), sind allerdings leichtflüchtige und geruchsintensive sowie ebenfalls gesundheitsschädliche und umweltgefährdende Verbindungen. Ausserdem neigen sie dazu, aufgrund geringer Verträglichkeit in der Zusammensetzung zu migrieren und dadurch Separation, Ausschwitzen oder Substratverschmutzung zu verursachen. Die beschriebene Verwendung von aromatischen, bei Raumtemperatur festen Amidinen und Guanidinen schafft hier Abhilfe, erfordert aber den Einsatz von geeigneten Lösemitteln und bringt Einbussen bei der katalytischen Aktivität und damit der Vernetzungsgeschwindigkeit

Das Dokument EP 2 100 923 offenbart die Verwendung von Guanidinderivate als Katalysatoren für die Aushärtung von Silangruppenhaltigen organischen Polymeren.

### Darstellung der Erfindung

Aufgabe der vorliegenden Erfindung ist es daher, eine bei Raumtemperatur härtbare Zusammensetzung auf Basis von Silangruppen-haltigen organischen Polymeren bereitzustellen, welche eine niedrige Gefahreneinstufung erlaubt, emissions- und geruchsarm ist, eine gute Lagerfähigkeit aufweist, mittels Feuchtigkeit unter Vernetzung der Silangruppen rasch aushärtet und dabei ein mechanisch hochwertiges und beständiges Material bildet, welches kaum zu migrationsbedingten Fehlern wie Ausschwitzen und Substratverschmutzung neigt.

Diese Aufgabe wird durch eine Zusammensetzung nach Anspruch 1 enthaltend einen Katalysator der Formel (I) gelöst. Diese Eigenschaften werden auch ohne die Verwendung von Metall-haltigen Verbindungen als Co-Katalysatoren erreicht, insbesondere ohne oder mit einer reduzierten Menge an Organozinn-Verbindungen. Der Katalysator der Formel (I) enthält eine aliphatische Guanidin-Gruppe und zeigt eine hohe katalytische Aktivität, während aromatische Guanidine kaum oder gar nicht katalytisch aktiv sind. Überraschenderweise weisen erfindungsgemässe Zusammensetzungen eine sehr schnelle, auf die Gegenwart des Katalysators der Formel (I) zurückgehende Aushärtung auf. Die Aktivität des Katalysators der Formel (I) ist überraschenderweise deutlich höher als diejenige vergleichbarer aliphatischer Guanidine wie beispielsweise 1,1,3,3-Tetramethylguanidin. Weiterhin überraschend ist die Tatsache, dass erfindungsgemässe Zusammensetzungen weder vor noch nach der Aushärtung zu migrationsbedingten Fehlern wie Separation, Ausschwitzen oder Substratverschmutzung neigen, im Gegensatz zu vielen ähnlichen Zusammensetzungen mit Amidin- und/oder Guanidin-Katalysatoren nach dem Stand der Technik, wo Katalysator-bedingte Migrationseffekte eine starke Rolle spielen.

Weiterhin überraschend ist die Tatsache, dass sich der Katalysator der Formel (I) in Gegenwart des Silangruppen-haltigen organischen Polymers, d.h. in der härtbaren Zusammensetzung selbst, ohne Störung des Systems herstellen lässt.

Weitere Aspekte der Erfindung sind Gegenstand weiterer unabhängiger Ansprüche. Besonders bevorzugte Ausführungsformen der Erfindung sind Gegenstand der abhängigen Ansprüche.

### Wege zur Ausführung der Erfindung

Die vorliegende Erfindung betrifft eine Zusammensetzung umfassend
- mindestens ein Silangruppen-haltiges organisches Polymer; und
- mindestens einen Katalysator der Formel (I), 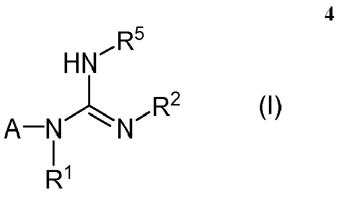
wobei
A für einen aliphatischen oder cycloaliphatischen oder arylaliphatischen Kohlenwasserstoff-Rest mit 3 bis 30 C-Atomen, welcher gegebenenfalls ungesättigte Anteile enthält, gegebenenfalls Sauerstoff- oder Stickstoffatome enthält und gegebenenfalls Aminogruppen oder Hydroxylgruppen aufweist, oder zusammen mit R¹ für einen zweiwertigen, gegebenenfalls verzweigten, Alkylen-Rest mit 4 bis 12, insbesondere 4 bis 8, C-Atomen, welcher gegebenenfalls ein Heteroatom enthält, steht,
R¹ für Wasserstoff oder für einen Alkyl- oder Cycloalkyl- oder Aralkyl-Rest mit 1 bis 8, insbesondere 1 bis 4, C-Atomen, oder zusammen mit A für einen zweiwertigen, gegebenenfalls verzweigten, Alkylen-Rest mit 4 bis 12, insbesondere 4 bis 8, C-Atomen, welcher gegebenenfalls ein Heteroatom enthält, steht, und R² und R⁵ unabhängig voneinander jeweils für Ethyl, Isopropyl, tert.Butyl, 3-(Dimethylamino)propyl oder Cyclohexyl stehen, und A nicht für den gleichen Rest wie R² und R⁵ steht;
wobei der Katalysator der Formel (I) kein Stickstoffatom enthält, welches direkt an einen aromatischen Ring gebunden oder Teil eines heteroaromatischen Ringsystems, wie zum Beispiel Imidazol oder Pyrimidin, ist.

Im vorliegenden Dokument bezeichnet der Begriff "Alkoxysilangruppe" oder kurz "Silangruppe" eine an einen organischen Rest gebundene Silylgruppe mit einem bis drei, insbesondere zwei oder drei, hydrolysierbaren Alkoxy-Resten am Silicium-Atom.

Entsprechend bezeichnet der Begriff "Alkoxysilan" oder kurz "Silan" eine organische Verbindung, welche mindestens eine Silangruppe aufweist.

Als "Hydroxysilan", "Isocyanatosilan", "Aminosilan" bzw. "Mercaptosilan" werden Silane bezeichnet, die am organischen Rest zusätzlich zur Silangruppe eine oder mehrere Hydroxyl-, Isocyanato-, Amino- bzw. Mercaptogruppen aufweisen.

Mit "Poly" beginnende Substanznamen wie Polyol oder Polyisocyanat bezeichnen Substanzen, die formal zwei oder mehr der in ihrem Namen vorkommenden funktionellen Gruppen pro Molekül enthalten.

Der Begriff "organisches Polymer" umfasst ein Kollektiv von chemisch einheitlichen, sich aber in Bezug auf Polymerisationsgrad, Molmasse und Kettenlänge unterscheidenden Makromolekülen, das durch eine Polyreaktion (Polymerisation, Polyaddition, Polykondensation) hergestellt wurde und im PolymerRückgrat mehrheitlich Kohlenstoffatome aufweist, sowie Umsetzungsprodukte eines solchen Kollektivs von Makromolekülen. Polymere mit einem Polydiorganosiloxan-Rückgrat (gemeinhin als "Silicone" bezeichnet) stellen keine organischen Polymere im Sinne des vorliegenden Dokuments dar.

Der Begriff "Silangruppen-haltiger Polyether" umfasst auch Silangruppen-haltige organische Polymere, welche zusätzlich zu Polyether-Einheiten auch Urethangruppen, Harnstoffgruppen oder Thiourethangruppen enthalten können. Solche Silangruppen-haltige Polyether können auch als "Silangruppen-haltige Polyurethane" bezeichnet werden.

Unter "Molekulargewicht" versteht man im vorliegenden Dokument die molare Masse (in Gramm pro Mol) eines Moleküls oder eines Teils eines Moleküls, auch als "Rest" bezeichnet. Als "mittleres Molekulargewicht" wird das Zahlenmittel Mₙ einer oligomeren oder polymeren Mischung von Molekülen oder Resten bezeichnet, welches üblicherweise mittels Gelpermeationschromatographie (GPC) gegen Polystyrol als Standard bestimmt wird.

Als "lagerstabil" oder "lagerfähig" wird eine Substanz oder eine Zusammensetzung bezeichnet, wenn sie bei Raumtemperatur in einem geeigneten Gebinde während längerer Zeit, typischerweise mindestens 3 Monaten bis zu 6 Monaten und mehr, aufbewahrt werden kann, ohne dass sie sich in ihren Anwendungs- oder Gebrauchseigenschaften, insbesondere der Viskosität und der Vernetzungsgeschwindigkeit, durch die Lagerung in einem für ihren Gebrauch relevanten Ausmass verändert.

Eine gestrichelte Linie in den Formeln in diesem Dokument stellt jeweils die Bindung zwischen einem Substituenten und dem zugehörigen Molekülrest dar. Als "Raumtemperatur" wird eine Temperatur von ca. 23 °C bezeichnet.

Der Katalysator der Formel (I) kann auch in tautomerer Form vorliegen. Alle möglichen Tautomerformen des Katalysators der Formel (I) werden im Rahmen der vorliegenden Erfindung als gleichwertig angesehen.

Weiterhin kann der Katalysator der Formel (I) in protonierter Form vorliegen. Ebenfalls kann der Katalysator der Formel (I) in komplexierter Form vorliegen, insbesondere mit Kationen von Zink, Eisen oder Molybdän.

Im Katalysator der Formel (I) steht A für einen aliphatischen oder cycloaliphatischen oder arylaliphatischen Kohlenwasserstoff-Rest mit 3 bis 30 C-Atomen, welcher gegebenenfalls ungesättigte Anteile enthält, gegebenenfalls Heteroatome, insbesondere Sauerstoff- oder Stickstoffatome, enthält und gegebenenfalls Aminogruppen oder Hydroxylgruppen oder Silangruppen aufweist.

Besonders bevorzugt ist A ausgewählt aus der Gruppe bestehend aus n-Hexyl, n-Octyl, 2-Ethylhexyl, n-Decyl, Lauryl, Cyclohexyl, Benzyl, 2-Methoxyethyl, 3-Methoxypropyl, Polyoxyalkylen-Rest mit Oxyethylen- und 1,2-Oxypropyleneinheiten und einem Molekulargewicht im Bereich von etwa 180 bis 600 g/mol, N-Methyl-3-aminopropyl, N-(2-Ethylhexyl)-3-aminopropyl, N-Cyclohexyl-3-aminopropyl, 3-(N,N-Dimethylamino)propyl, 2-Aminopropyl, 3-Aminopropyl, 3-Aminopentyl, 5-Amino-4-methylpentyl, 5-Amino-2-methylpentyl, 6-Aminohexyl, 6-Amino-3,3(5),5-trimethylhexyl, 6-Amino-2,2(4),4-trimethylhexyl, 8-Aminooctyl, 10-Aminodecyl, 12-Aminododecyl, 3-Aminomethyl-3,5,5-trimethylcyclohexyl, 3-Amino-1,5,5-trimethylcyclohexylmethyl, 3-Aminomethyl-cyclohexylmethyl, 4-Aminomethyl-cyclohexylmethyl, 3-Aminomethyl-benzyl, 5-Amino-3-oxa-pentyl, ω -2-Aminopropyl-polyoxypropylen-Rest mit einem Molekulargewicht im Bereich von etwa 200 bis 500 g/mol, 2-Hydroxypropyl, 3-Hydroxypropyl, 1,1-Dimethyl-2-hydroxyethyl, und 5-Hydroxy-3-oxa-pentyl.

In einer Ausführungsform der Erfindung sind diejenigen Reste A bevorzugt, welche einen Kohlenwasserstoffrest mit 6 bis 30 C-Atomen, welcher aussser gegebenenfalls Ethersauerstoffen keine weiteren funktionellen Gruppen aufweist, darstellen, also insbesondere Hexyl, Octyl, 2-Ethylhexyl, Decyl, Lauryl, Cyclohexyl, Benzyl, 2-Methoxyethyl, 3-Methoxypropyl oder einen Polyoxyalkylen-Rest mit Oxyethylen- und 1,2-Oxypropyleneinheiten und einem Molekulargewicht im Bereich von etwa 180 bis 600 g/mol. Ein solcher Katalysator der Formel (I) ist nicht oder wenig feuchtigkeitsempfindlich und ist aus kostengünstigen Ausgangsstoffen besonders einfach und in hoher Reinheit herstellbar. Überraschenderweise migriert ein solcher Silangruppen-freier Katalysator nicht an die Oberfläche des ausgehärteten Polymers, obwohl er bei der Aushärtung nicht ins organische Polymer eingebaut werden kann.

In einer weiteren Ausführungsform der Erfindung sind diejenigen Reste A bevorzugt, welche einen Silangruppen-freien Kohlenwasserstoffrest mit 3 bis 30 C-Atomen mit gegebenenfalls Ethersauerstoffen und mit zusätzlich mindestens einer Hydroxylgruppe oder primären oder sekundären Aminogruppe darstellen, also insbesondere N-Methyl-3-aminopropyl, N-(2-Ethylhexyl)-3-aminopropyl, N-Cyclohexyl-3-aminopropyl, 2-Aminopropyl, 3-Aminopropyl, 3-Aminopentyl, 5-Amino-4-methylpentyl, 5-Amino-2-methylpentyl, 6-Aminohexyl, 6-Amino-3,3(5),5-trimethylhexyl, 6-Amino-2,2(4),4-trimethylhexyl, 8-Aminooctyl, 10-Aminodecyl, 12-Aminododecyl, 3-Aminomethyl-3,5,5-trimethylcyclohexyl, 3-Amino-1,5,5-trimethylcyclohexylmethyl, 3-Aminomethyl-cyclohexylmethyl, 4-Aminomethyl-cyclohexylmethyl, 3-Aminomethyl-benzyl, 5-Amino-3-oxa-pentyl, ω-2-Aminopropyl-polyoxypropylen-Rest mit einem Molekulargewicht im Bereich von 200 bis 500 g/mol, 2-Hydroxypropyl, 3-Hydroxypropyl, 1,1-Dimethyl-2-hydroxyethyl oder 5-Hydroxy-3-oxa-pentyl. Ein solcher Katalysator der Formel (I) ist nicht oder wenig feuchtigkeitsempfindlich und geruchsarm. Überraschenderweise ist er besonders schwerflüchtig. Aufgrund der Hydroxylgruppe bzw. der primären oder sekundären Aminogruppe kann er über eine besonders hohe katalytische Aktivität verfügen oder eine besonders gute Wechselwirkung mit dem Silangruppen-haltigen Polymer aufweisen. Überraschenderweise migriert ein solcher Silangruppen-freier Katalysator nicht an die Oberfläche des ausgehärteten Polymers, obwohl er bei der Aushärtung nicht ins organische Polymer eingebaut werden kann.

Davon bevorzugt sind Reste A, welche eine primäre oder sekundäre Aminogruppe aufweisen. Diese Katalysatoren der Formel (I) weisen eine besonders hohe katalytische Aktivität auf.

In einer weiteren Ausführungsform der Erfindung sind diejenigen Reste A bevorzugt, welche einen Silangruppen-freien Kohlenwasserstoffrest mit 3 bis 30 C-Atomen mit gegebenenfalls Ethersauerstoff und mit zusätzlich mindestens einer tertiären Aminogruppe darstellen, insbesondere 3-(N,N-Dimethylamino)-propyl. Ein solcher Katalysator der Formel (I) weist eine besonders hohe katalytische Aktivität auf. Zudem ist er nicht oder wenig feuchtigkeitsempfindlich und geruchsarm. Überraschenderweise migriert ein solcher Silangruppen-freier Katalysator nicht an die Oberfläche des ausgehärteten Polymers, obwohl er bei der Aushärtung nicht ins organische Polymer eingebaut werden kann.

A steht nicht für den gleichen Rest wie R² und R⁵. Insbesondere steht A nicht für einen Cyclohexyl-Rest, wenn R² und R⁵ für je einen Cyclohexyl-Rest stehen. Katalysatoren der Formel (I) mit den gleichen Resten für A, R² und R⁵ neigen aufgrund der hohen Symmetrie zum Kristallisieren, was ihre Verarbeitung erschwert.

Besonders bevorzugt steht R¹ für Wasserstoff. Diese Katalysatoren der Formel (I) sind besonders gut herstellbar und besonders aktiv.

R² und R⁵ stehen unabhängig voneinander jeweils für Ethyl, Isopropyl, tert.Butyl, 3-(Dimethylamino)propyl oder Cyclohexyl, insbesondere für Isopropyl oder Cyclohexyl, am meisten bevorzugt für Cyclohexyl.

Die bevorzugten Ausführungsformen des Katalysators lassen sich aus handelsüblichen, preisgünstigen Rohstoffen herstellen und haben besonders gute Eigenschaften in Bezug auf katalytische Aktivität und Verträglichkeit des Katalysators.

Bevorzugt sind Katalysatoren der Formel (I), welche bei Raumtemperatur in flüssiger Form vorliegen. Solche Katalysatoren lassen sich einfach fördern, dosieren und gut in eine Zusammensetzung einarbeiten, während Feststoffe typischerweise ein Lösemittel erfordern.

Das Gewichtsverhältnis zwischen dem Silangruppen-haltigen organischen Polymer und dem Katalysator der Formel (I) beträgt bevorzugt mindestens 10/1, insbesondere mindestens 20/1, besonders bevorzugt mindestens 40/1. Das Gewichtsverhältnis zwischen dem Silangruppen-haltigen organischen Polymer und dem Katalysator der Formel (I) beträgt bevorzugt höchstens 10'000/1, insbesondere höchstens 2'000/1, besonders bevorzugt höchstens 1'000/1 .

Bevorzugt liegt das Gewichtsverhältnis zwischen dem Silangruppen-haltigen organischen Polymer und dem Katalysator der Formel (I) im Bereich von 10/1 bis 2'000/1, besonders bevorzugt 20/1 bis 1'000/1, insbesondere 40/1 bis 1'000/1 .

Eine solche Zusammensetzung weist eine gute Lagerfähigkeit und eine schnelle Aushärtung auf.

Der Katalysator der Formel (I) wird insbesondere erhalten durch die Umsetzung von mindestens einem Amin der Formel (II) mit mindestens einem Carbodiimid der Formel (III).

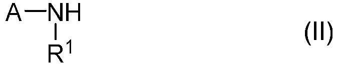

R⁵-N=C=N-R² (III)

A, R¹, R² und R⁵ wurden bereits vorhergehend beschrieben.

Die Umsetzung des Amins mit dem Carbodiimid wird bevorzugt bei erhöhter Temperatur, insbesondere bei 40 bis 160 °C und besonders bevorzugt bei 60 bis 140 °C durchgeführt. Die Umsetzung kann ganz ohne die Verwendung von VOC-Lösemitteln erfolgen. Zur Beschleunigung der Umsetzung kann gegebenenfalls ein Katalysator eingesetzt werden, insbesondere eine Säure wie z.B. eine Carbonsäure oder Kohlensäure, oder eine Lewis-Säure wie z.B. Bortrifluorid-Etherat, Aluminiumchlorid, Aluminiumacetylacetonat, Eisen(III)chlorid, Lithiumperchlorate, Zinkchlorid, Zinkacetat, Zink neodecanoat, Zinkacetylacetonat, Zinktriflat oder Lanthantriflat.
Die Umsetzung kann einstufig oder mehrstufig erfolgen.
Die Umsetzung erfolgt geeigneterweise unterstöchiometrisch bis stöchiometrisch in Bezug auf das Carbodiimid der Formel (III), bevorzugt in einem solchen Verhältnis, dass pro mol Amin der Formel (II) 0.1 bis 1.0 mol Carbodiimid der Formel (III) eingesetzt werden. Es wird also nur höchstens eine Aminogruppe pro Amin der Formel (II) umgesetzt, auch wenn das Amin der Formel (II) mehrere Aminogruppen aufweist.
Das Reaktionsprodukt aus diesem Verfahren wird bevorzugt ohne Aufarbeitung und/oder Reinigung in der Zusammensetzung verwendet, mit Ausnahme der destillativen Entfernung von flüchtigen Verbindungen, gegebenenfalls unter Vakuum.

Das Amin der Formel (II) ist typischerweise ein aliphatisches, cycloaliphatisches oder arylaliphatisches Amin mit mindestens einer primären oder sekundären Aminogruppe, insbesondere ausgewählt aus der folgenden Gruppe:
- aliphatische, cycloaliphatische oder arylaliphatische gegebenenfalls Ethergruppen aufweisende Monoamine, insbesondere Methylamin, Ethylamin, Propylamin, Isopropylamin, n-Butylamin, Isobutylamin, sec.Butylamin, tert.Butylamin, n-Pentylamin, Isopentylamin, 3-Methyl-2-butylamin, n-Hexylamin, n-Octylamin, 2-Ethylhexylamin, n-Decylamin, Laurylamin, Myristylamin, Palmitylamin, Stearylamin, Cyclohexylamin, Benzylamin, sowie von natürlichen Fettsäuregemischen abgeleitete Fettamine, wie zum Beispiel Cocoalkylamin, C₁₆-C₂₂-Alkylamin, Soyaalkylamin, Oleylamin und Talgalkylamin, erhältlich beispielsweise unter den Handelsnamen Armeen® (von Akzo Nobel) oder Rofamin® (von Ecogreen Oleochemicals), 2-Methoxyethylamin, 2-Ethoxyethylamin, 2-Butoxyethylamin, 2-Cyclohexyloxyethylamin, 2-Benzyloxyethylamin, 3-Methoxypropylamin, 3-Ethoxypropylamin, 3-Butoxypropylamin, 3-Hexyloxypropylamin, 3-(2-Ethylhexyloxy)propylamin, 3-Cyclohexyloxypropylamin, 3-Phenyloxypropylamin, 3-(2-Methoxyethoxy)propylamin, 2(4)-Methoxyphenylethylamin, Morpholin, 2,6-Dimethylmorpholin und 2-Aminoethylmorpholin, sowie Polyetheramine, insbesondere Polyoxyalkylenamine, insbesondere im Handel erhältliche Typen wie insbesondere Jeffamine® XTJ-581, Jeffamine® M-600, Jeffamine® M-1000, Jeffamine® M-2005, Jeffamine® M-2070 (alle von Huntsman), sowie Amine von Fettalkohol- oder Alkylphenolalkoxylaten wie insbesondere Jeffamine® XTJ-247, Jeffamine® XTJ-248, Jeffamine® XTJ-249, Jeffamine® XTJ-435 und Jeffamine® XTJ-436 (alle von Huntsman), Dimethylamin, Diethylamin, Dipropylamin, Diisopropylamin, Dibutylamin, Dihexylamin, Di-(2-ethylhexyl)amin, Dicyclohexylamin, Dibenzylamin, Methylbutylamin, Methylcyclohexylamin, Methylbenzylamin, Pyrrolidin, Piperidin, 3,5-Dimethylpiperidin, N-Methylpiperazin und N-Ethylpiperazin;
- aliphatische, cycloaliphatische oder arylaliphatische Polyamine und Hydroxylamine, insbesondere Ethylendiamin, 1,2- und 1,3-Propandiamin, 2-Methyl-1,2-propandiamin, 2,2-Dimethyl-1,3-propandiamin, 1,3- und 1,4-Butandiamin, 1,3-Pentandiamin (DAMP), 1,5-Pentandiamin, 1,5-Diamino-2-methylpentan (MPMD), 2-Butyl-2-ethyl-1,5-pentandiamin (C11-Neodiamin), 1,6-Hexandiamin, 2,5-Dimethyl-1,6-hexandiamin, 2,2,4- und 2,4,4-Trimethylhexamethylendiamin (TMD), 1,7-Heptandiamin, 1,8-Octandiamin, 1,9-Nonandiamin, 1,10-Decandiamin, 1,11-Undecandiamin, 1,12-Dodecandiamin, 1,2-, 1,3- und 1,4-Diaminocyclohexan, Bis(4-aminocyclohexyl)methan, Bis-(4-amino-3-methylcyclohexyl)methan, Bis(4-amino-3-ethylcyclohexyl)methan, Bis(4-amino-3,5-dimethylcyclohexyl)methan, Bis(4-amino-3-ethyl-5-methylcyclohexyl)methan, 1-Amino-3-aminomethyl-3,5,5-trimethylcyclohexan (= Isophorondiamin oder IPD), 2- und 4-Methyl-1,3-diaminocyclohexan und Mischungen davon, 1,3- und 1,4-Bis(aminomethyl)cyclohexan, 2,5(2,6)-Bis(aminomethyl)bicyclo[2.2.1]heptan (NBDA), 3(4), 8(9)-Bis(aminomethyl)-tricyclo[5.2.1.02,6]decan, 1,4-Diamino-2,2,6-trimethylcyclohexan (TMCDA), 1,8-Menthandiamin, 3,9-Bis(3-aminopropyl)-2,4,8,10-tetraoxaspiro[5.5]undecan, 1,3-Bis(aminomethyl)benzol, 1,4-Bis(aminomethyl)benzol, N-Methyl-1,2-ethandiamin, N-Ethyl-1,2-ethandiamin, N-Butyl-1,2-ethandiamin, N-Hexyl-1,2-ethandiamin, N-(2-Ethylhexyl)-1,2-ethandiamin, N-Cyclohexyl-1,2-ethandiamin, 4-Aminomethyl-piperidin, 3-(4-Aminobutyl)-piperidin, N-Methyl-1,3-propandiamin, N-Ethyl-1,3-propandiamin, N-Butyl-1,3-propandiamin, N-Hexyl-1,3-propandiamin, N-(2-Ethylhexyl)-1,3-propandiamin, N-Dodecyl-1,3-propandiamin, N-Cyclohexyl-1,3-propandiamin, 3-Methylamino-1-pentylamin, 3-Ethylamino-1-pentylamin, 3-Butylamino-1-pentylamin, 3-Hexylamino-1-pentylamin, 3-(2-Ethylhexyl)amino-1-pentylamin, 3-Dodecylamino-1-pentylamin, 3-Cyclohexylamino-1-pentylamin, N-(2-Aminoethyl)piperazin, N-(2-Aminopropyl)piperazin, N¹-((3-Dimethylamino)propyl)-1,3-diaminopropan, sowie 3-aminopropylierte Fettamine wie insbesondere N-Cocoalkyl-1,3-propandiamin, N-Oleyl-1,3-propandiamin, N-Soyaalkyl-1,3-propandiamin, N-Talgalkyl-1,3-propandiamin und N-(C₁₆₋₂₂-Alkyl)-1,3-propandiamin, wie sie beispielsweise unter dem Handelsnamen Duomeen® (von Akzo Nobel) erhältlich sind, N,N-Dimethyl-1,2-ethandiamin, N,N-Diethyl-1,2-ethandiamin, N,N-Dimethyl-1,3-propandiamin, N,N-Diethyl-1,3-propandiamin, N¹,N¹-Diethyl-1,4-pentandiamin, 2-Aminoethyl-piperidin, 2-Aminopropyl-piperidin, 2-Morpholino-ethylamin, 3-Morpholino-propylamin, N,N'-Bis(aminopropyl)-piperazin, N,N-Bis(3-aminopropyl)methylamin, N,N-Bis(3-aminopropyl)ethylamin, N,N-Bis(3-aminopropyl)propylamin, N,N-Bis(3-aminopropyl)cyclohexylamin, N,N-Bis(3-aminopropyl)-2-ethyl-hexylamin, Produkte aus der doppelten Cyanoethylierung und nachfolgender Reduktion von Fettaminen, welche abgeleitet sind von natürlichen Fettsäuren, wie N,N-Bis(3-aminopropyl)dodecylamin und N,N-Bis(3-aminopropyl)talgalkylamin, erhältlich als Triameen® Y12D und Triameen® YT (von Akzo Nobel), 4-Aminomethyl-1,8-octandiamin, 1,3,5-Tris-(aminomethyl)-cyclohexan, 1,3,5-Tris(aminomethyl)-benzol, Tris(2-aminoethyl)amin, Diethylentriamin (DETA), Triethylentetramin (TETA), Tetraethylenpentamin (TEPA), Pentaethylenhexamin (PEHA), Polyethylenpolyamin mit 5 bis 7 Ethylenamin-Einheiten (sogenanntes "higher ethylenepolyamine", HEPA), Dipropylentriamin (DPTA), N-(2-Aminoethyl)-1,3-propandiamin (N3-Amin), N,N'-Bis(3-aminopropyl)ethylendiamin (N4-Amin), Bis-hexamethylentriamin (BHMT), N3-(3-Aminopentyl)-1,3-pentandiamin, N5-(3-Aminopropyl)-2-methyl-1,5-pentandiamin und N5-(3-Amino-1-ethylpropyl)-2-methyl-1,5-pentandiamin, Bis(2-aminoethyl)ether, 3,6-Dioxa-octan-1,8-diamin, 4,7-Dioxadecan-1,10-diamin, 4,7-Dioxadecan-2,9-diamin, 4,9-Dioxadodecan-1,12-diamin, 5,8-Dioxadodecan-3,10-diamin, 4,7,10-Tri-oxatridecan-1,13-diamin, cycloaliphatische ethergruppenhaltige Diamine aus der Propoxylierung und nachfolgenden Aminierung von 1,4-Dimethylolcyclohexan, erhältlich insbesondere als Jeffamine® RFD-270 (von Huntsman), Polyoxyalkylenamine mit einem mittleren Molekulargewicht im Bereich von 200 bis 2000 g/mol, wie sie im Handel beispielsweise unter den Handelsnamen Jeffamine® (von Huntsman), Polyetheramine (von BASF) und PC Amine® (von Nitroil) erhältlich sind, dadurch gekennzeichnet, dass sie 2-Aminopropyl- oder 2-Aminobutyl-Endgruppen tragen, insbesondere Jeffamine® D-230, Jeffamine® D-400, Jeffamine® D-2000, Jeffamine® XTJ-582, Jeffamine® XTJ-578, Jeffamine® HK-511, Jeffamine® ED-600, Jeffamine® ED-900, Jeffamine® ED-2003, , Jeffamine® XTJ-569, Jeffamine® XTJ-533, Jeffamine® XTJ-536, Jeffamine® THF-100, Jeffamine® THF-170, Jeffamine® THF-140, Jeffamine® THF-230, Jeffamine® SD-231, Jeffamine® SD-401, Jeffamine® SD-2001, Jeffamine® T-403, Jeffamine® XTJ-566 und Jeffamine® ST-404 (alle von Huntsman), sowie dazu analoge Typen von BASF und Nitroil, aminopropylierte Polyoxyalkylenamine, wie sie durch Umsetzen von Polyoxyalkylenaminen mit Acrylnitril und nachfolgender Hydrierung erhältlich sind, Polyethylenimine, insbesondere im Handel unter den Markennamen Lupasol® (von BASF) oder Epomin® (von Nippon Shokubai) erhältliche Typen wie Lupasol® FG, Lupasol® G 20 wasserfrei, Epomin® SP-003, Epomin® SP-006, Epomin® SP-012 und Epomin® SP-018, 2-Aminoethanol, 2-Methylaminoethanol (2-Amino-1-propanol), 1-Amino-2-propanol, 3-Amino-1-propanol, 4-Amino-1-butanol, 4-Amino-2-butanol, 2-Amino-2-methylpropanol, 5-Amino-1-pentanol, 6-Amino-1-hexanol, 7-Amino-1-heptanol, 8-Amino-1-octanol, 10-Amino-1-decanol, 12-Amino-1-dodecanol, 4-(2-Aminoethyl)-2-hydroxyethylbenzol, 3-Aminomethyl-3,5,5-trimethyl-cyclohexanol; eine primäre Aminogruppe tragende Derivate von Glykolen wie Diethylenglykol, Dipropylenglykol, Dibutylenglykol und höheren Oligomeren und Polymeren dieser Glykole, insbesondere 2-(2-Aminoethoxy)ethanol, 2-(2-(2-Amino-ethoxy)ethoxy)ethanol, alpha-(2-Hydroxymethylethyl)-ω-(2-aminomethyl-ethoxy)poly(oxy(methyl-1,2-ethandiyl)), eine Hydroxylgruppe und eine primäre Aminogruppe tragende Derivate von polyalkoxylierten drei- oder höherwertigen Alkoholen, Produkte aus der einfachen Cyanoethylierung und anschliessender Hydrierung von Glykolen, insbesondere 3-(2-Hydroxyethoxy)propylamin, 3-(2-(2-Hydroxyethoxy)ethoxy)propylamin und 3-(6-Hydroxyhexyloxy)propylamin;
- Davon bevorzugt sind Amine mit mindestens einer primären Aminogruppe. Davon bevorzugt sind weiterhin Amine mit 2 bis zu 50 C-Atomen, besonders bevorzugt sind Amine mit 3 bis zu 30 C-Atomen.

Besonders bevorzugte Amine sind ausgewählt aus der Gruppe bestehend aus n-Hexylamin, n-Octylamin, 2-Ethylhexylamin, n-Decylamin, Laurylamin, Cyclohexylamin, Benzylamin, 2-Methoxyethylamin, 3-Methoxypropylamin, Polyoxyalkylenamin mit Oxyethylen- und 1,2-Oxypropyleneinheiten und einem mittleren Molekulargeicht im Bereich von 180 bis 600 g/mol, insbesondere Jeffamine® M-600, N-Methyl-1,3-propandiamin, N-(2-Ethylhexyl)-1,3-propandiamin, N-Cyclohexyl-1,3-propandiamin, N,N-Dimethyl-1,3-propandiamin, 1,2-Propandiamin, 1,3-Propandiamin, 1,3-Pentandiamin (DAMP), 1,5-Diamino-2-methylpentan (MPMD), 1,6-Hexandiamin, 2,2,4- und 2,4,4-Trimethylhexamethylendiamin (TMD), 1,8-Octandiamin, 1,10-Decandiamin, 1,12-Dodecandiamin, 1-Amino-3-aminomethyl-3,5,5-trimethylcyclohexan (= Isophorondiamin oder IPD), 1,3-Bis-(aminomethyl)cyclohexan, 1,4-Bis(aminomethyl)cyclohexan,1,3-Bis(aminomethyl)benzol, Bis-(2-aminoethyl)ether, Polyoxypropylendiamin mit einem mittleren Molekulargewicht im Bereich von etwa 220 bis 500 g/mol, insbesondere Jeffamine® D-230 und Jeffamine® D-400, 1-Amino-2-propanol, 3-Amino-1-propanol, 2-Amino-2-methyl-1-propanol, und 2-(2-Aminoethoxy)ethanol.

Davon bevorzugt sind primäre Monoamine ohne Silan- oder Hydroxylgruppen mit 6 bis 30 C-Atomen, also insbesondere n-Hexylamin, n-Octylamin, 2-Ethylhexylamin, n-Decylamin, Laurylamin, Cyclohexylamin, Benzylamin, 2-Methoxyethylamin, 3-Methoxypropylamin und Polyoxyalkylenamin mit Oxyethylen- und 1,2-Oxypropyleneinheiten und einem Molekulargeicht im Bereich von 180 bis 600 g/mol, insbesondere Jeffamine® M-600. Mit diesen Aminen sind Katalysatoren der Formel (I) erhältlich, welche nicht oder wenig feuchtigkeitsempfindlich und aus kostengünstigen Ausgangsstoffen besonders einfach und in hoher Reinheit herstellbar sind.

Davon weiterhin bevorzugt sind Silangruppen-freie Polyamine und Hydroxylamine mit 3 bis 30 C-Atomen, insbesondere N-Methyl-1,3-propandiamin, N-(2-Ethylhexyl)-1,3-propandiamin, N-Cyclohexyl-1,3-propandiamin, 1,2-Propandiamin, 1,3-Propandiamin, 1,3-Pentandiamin (DAMP), 1,5-Diamino-2-methylpentan (MPMD), 1,6-Hexandiamin, 2,2(4),4-Trimethylhexamethylendiamin (TMD), 1,8-Octandiamin, 1,10-Decandiamin, 1,12-Dodecandiamin, 1-Amino-3-aminomethyl-3,5,5-trimethylcyclohexan (= Isophorondiamin oder IPD), 1,3-Bis(aminomethyl)cyclohexan, 1,4-Bis(aminomethyl)cyclohexan,1,3-Bis(aminomethyl)-benzol, Bis-(2-aminoethyl)ether, Polyoxypropylendiamin mit einem Molekulargewicht im Bereich von etwa 220 bis 500 g/mol, insbesondere Jeffamine® D-230 und Jeffamine® D-400, 1-Amino-2-propanol, 3-Amino-1-propanol, 2-Amino-2-methyl-1-propanol und 2-(2-Aminoethoxy)ethanol. Mit diesen Aminen sind überraschend schwerflüchtige Katalysatoren der Formel (I) erhältlich, welche nicht oder wenig feuchtigkeitsempfindlich und geruchsarm sind und durch die Hydroxyl- oder Aminogruppen zusätzliche Effekte wie eine ganz besonders hohe Aktivität oder eine besonders gute Wechselwirkung mit dem Silangruppen-haltigen Polymer aufweisen können.

Davon weiterhin bevorzugt sind Silangruppen-freie Polyamine mit 3 bis 30 C-Atomen, welche mindestens eine tertiäre Aminogruppe aufweisen, insbesondere N,N-Dimethyl-1,3-propandiamin. Mit diesen Aminen sind überraschend aktive Katalysatoren der Formel (I) erhältlich, welche nicht oder wenig feuchtigkeitsempfindlich und geruchsarm sind.

Als Carbodiimid eignen sich typischerweise aliphatische, cycloaliphatische oder arylaliphatische Carbodiimide, insbesondere einfache, im Handel erhältliche aliphatische und cycloaliphatische Carbodiimide.

Bevorzugt ist das Carbodiimid der Formel (III) ausgewählt aus der Gruppe bestehend aus N,N'-Diisopropylcarbodiimid (DIC), N,N'-Di-tert.butylcarbodiimid, N,N'-Dicyclohexylcarbodiimid (DCC) und N-Ethyl-N'-(3-dimethylaminopropyl)-carbodiimid (EDC). Besonders bevorzugt ist N,N'-Diisopropylcarbodiimid (DIC) oder N,N'-Dicyclohexylcarbodiimid (DCC), insbesondere DCC.

In einer bevorzugten Ausführungsform wird der Katalysator der Formel (I) "in situ", d.h. in Gegenwart des Silangruppen-haltigen organischen Polymers, hergestellt. Dazu werden das Amin der Formel (II) und das Carbodiimid der Formel (III) mit dem Silangruppen-haltigen organischen Polymer vermischt und bei einer Temperatur im Bereich von 40 bis 120 °C umgesetzt. Die in situ-Umsetzung kann insbesondere auch in Gegenwart weiterer Inhaltsstoffe, wie sie für härtbare Zusammensetzungen auf Basis Silangruppen-haltiger organischer Polymere typisch sind, insbesondere in Gegenwart von Hilfs- und Zusatzstoffen wie weiter unten beschrieben, durchgeführt werden. Überraschenderweise kann die Umsetzung zum Katalysator der Formel (I) auch in situ in einem voll formulierten Dichtstoff oder Klebstoff oder einer voll formulierten Beschichtung basierend jeweils auf einem Silangruppen-haltigen organischen Polymer erfolgen, also auch in Gegenwart von beispielsweise Trocknungsmitteln und/oder Weichmachern und/oder Füllstoffen und/oder Rheologie-Modifizierern. Die insitu Herstellung des Katalysators der Formel (I) in der Zusammensetzung ist besonders bevorzugt, da der Katalysator somit erst mit einer gewissen zeitlichen Verzögerung entsteht und dadurch den Compoundierprozess wenig stört. Dies kann in der Praxis einen bedeutenden Vorteil darstellen, beispielsweise im Fall von feuchten Bestandteilen wie Füllstoffen, die so in der Zusammensetzung mit Trocknungsmitteln getrocknet werden können und deren Feuchtigkeit somit nicht unter Einbezug des Katalysators mit Silangruppen des Silangruppen-haltigen organischen Polymers reagieren. Dadurch wird die Viskosität der Zusammensetzung nicht unerwünscht erhöht.

Besonders bevorzugte Katalysatoren der Formel (I) sind ausgewählt aus der Gruppe bestehend aus 1-Hexyl-2,3-diisopropylguanidin, 1-Hexyl-2,3-dicyclohexylguanidin, 1-Octyl-2,3-diisopropylguanidin, 1-Octyl-2,3-dicyclohexylguanidin, 1-(2-Ethylhexyl)-2,3-diisopropylguanidin, 1-(2-Ethylhexyl)-2,3-dicyclohexylguanidin, 1-Decyl-2,3-diisopropylguanidin, 1-Decyl-2,3-dicyclohexylguanidin, 1-Lauryl-2,3-diisopropylguanidin, 1-Lauryl-2,3-dicyclohexylguanidin, 1-Cyclohexyl-2,3-diisopropylguanidin, 1,2,3-Tricyclohexylguanidin, 1-Benzyl-2,3-diisopropylguanidin, 1-Benzyl-2,3-dicyclohexylguanidin, 1-(2-Methoxyethyl)-2,3-diisopropylguanidin, 1-(2-Methoxyethyl)-2,3-dicyclohexylguanidin, 1-(2-Methoxypropyl)-2,3-diisopropylguanidin, 1-(2-Methoxypropyl)-2,3-dicyclohexylguanidin, 1-(ω-Methoxy-polyoxypropylen-polyoxyethylen)-2,3-diisopropylguanidin mit einem Molekulargewicht im Bereich von etwa 320 bis 750 g/mol, 1-(ω-Methο-xy-polyoxypropylen-polyoxyethylen)-2,3-dicyclohexylguanidin mit einem Molekulargewicht im Bereich von etwa 400 bis 830 g/mol, 1-(2-Aminopropyl)-2,3-diisopropylguanidin, 1-(2-Aminopropyl)-2,3-dicyclohexylguanidin, 1-(3-Aminopropyl)-2,3-diisopropylguanidin, 1-(3-Aminopropyl)-2,3-dicyclohexylguanidin, 1-(3-Methylaminopropyl)-2,3-diisopropylguanidin, 1-(3-Methylaminopropyl)-2,3-dicyclohexylguanidin, 1-(3-(2-Ethylhexylamino)propyl)-2,3-diisopropylguanidin, 1-(3-(2-Ethylhexylamino)propyl)-2,3-dicyclohexylguanidin, 1-(3-Cyclohexyl-aminopropyl)-2,3-diisopropylguanidin, 1-(3-Cyclohexylaminopropyl)-2,3-dicyclohexylguanidin, 1-(3-(N,N-Dimethylamino)propyl)-2,3-diisopropylguanidin, 1-(3-(N,N-Dimethylamino)propyl)-2,3-dicyclohexylguanidin, 1-(3-Aminopentyl)-2,3-diisopropylguanidin, 1-(3-Aminopentyl)-2,3-dicyclohexylguanidin, 1-(5-Amino-4-methylpentyl)-2,3-diisopropylguanidin, 1-(5-Amino-4-methylpentyl)-2,3-dicyclohexylguanidin, 1-(5-Amino-2-methylpentyl)-2,3-diisopropylguanidin, 1-(5-Amino-2-methylpentyl)-2,3-dicyclohexylguanidin, 1-(6-Aminohexyl)-2,3-diiso-propylguanidin, 1-(6-Aminohexyl)-2,3-dicyclohexylguanidin, 1-(6-Amino-2,2(4),4-trimethyl-hexyl)-2,3-diisopropylguanidin, 1-(6-Amino-2,2(4),4-trimethylhexyl)-2,3-dicyclohexylguanidin, 1-(6-Amino-3,3(5),5-trimethyl-hexyl)-2,3-diiso-propylguanidin, 1-(6-Amino-3,3(5),5-trimethyl-hexyl)-2,3-dicyclohexylguanidin, 1-(8-Aminooctyl)-2,3-diisopropylguanidin, 1-(8-Aminooctyl)-2,3-dicyclohexylguanidin, 1-(10-Aminodecyl)-2,3-diisopropylguanidin, 1-(10-Aminodecyl)-2,3-dicyclohexylguanidin, 1-(12-Aminododecyl)-2,3-diisopropylguanidin, 1-(12-Aminododecyl)-2,3-dicyclohexylguanidin, 1-(3-Aminomethyl-3,5,5-trimethyl-cyclohexyl)-2,3-diisopropylguanidin, 1-(3-Aminomethyl-3,5,5-trimethyl-cyclohexyl)-2,3-dicyclohexylguanidin, 1-(3-Amino-1,5,5-trimethyl-cyclohexylmethyl)-2,3-di-isopropylguanidin, 1-(3-Amino-1,5,5-trimethyl-cyclohexylmethyl)-2,3-dicyclohexylguanidin, 1-(3-Aminomethyl-cyclohexylmethyl)-2,3-diisopropylguanidin, 1-(3-Aminomethyl-cyclohexylmethyl)-2,3-dicyclohexylguanidin, 1-(4-Aminomethyl-cyclohexylmethyl)-2,3-diisopropylguanidin, 1-(4-Aminomethyl-cyclohexylmethyl)-2,3-dicyclohexylguanidin, 1-(3-Aminomethyl-benzyl)-2,3-diisopropylguanidin, 1-(3-Aminomethyl-benzyl)-2,3-dicyclohexylguanidin, 1-(5-Amino-3-oxa-pentyl)-2,3-diisopropylguanidin, 1-(5-Amino-3-oxa-pentyl)-2,3-dicyclo-hexylguanidin, 1-(ω-2-Aminopropyl-polyoxypropylen)-2,3-diisopropylguanidin mit einem Molekulargewicht im Bereich von etwa 340 bis 650 g/mol, 1-((Amino-polyoxypropylen)yl)-2,3-dicyclohexylguanidin mit einem Molekulargewicht im Bereich von etwa 420 bis 750 g/mol, 1-(2-Hydroxypropyl)-2,3-diisopropylguanidin, 1-(2-Hydroxypropyl)-2,3-dicyclohexylguanidin, 1-(3-Hydroxypropyl)-2,3-di-isopropylguanidin, 1-(3-Hydroxypropyl)-2,3-dicyclohexylguanidin, 1-(2-Hydroxy-1,1-dimethyl-ethyl)-2,3-diisopropylguanidin, 1-(2-Hydroxy-1,1-dimethyl-ethyl)-2,3-dicyclohexylguanidin, 1-(5-Hydroxy-3-oxa-pentyl)-2,3-diisopropylguanidin, 1-(5-Hydroxy-3-oxa-pentyl)-2,3-dicyclohexylguanidin.

Davon bevorzugt sind Guanidin ohne Silangruppen, ohne Aminogruppen und ohne Hydroxylgruppen. Diese Guanidine sind nicht oder wenig feuchtigkeitsempfindlich und lassen sich besonders rein herstellen.
Davon weiterhin bevorzugt sind primäre oder sekundäre Aminogruppen oder Hydroxylgruppen aufweisende Guanidine ohne Silangruppen. Diese Guanidine sind überraschend schwerflüchtig, nicht oder wenig feuchtigkeitsempfindlich und geruchsarm und können durch die Hydroxyl- oder Aminogruppen zusätzliche Effekte wie eine ganz besonders hohe Aktivität oder eine besonders gute Wechselwirkung mit dem Silangruppen-haltigen Polymer aufweisen.
Davon weiterhin bevorzugt sind tertiäre Aminogruppen aufweisende Guanidine ohne Silangruppen. Diese Guanidine weisen eine überrraschend hohe katalytische Aktivität auf und sind nicht oder wenig feuchtigkeitsempfindlich und geruchsarm.
Davon weiterhin bevorzugt sind Silangruppen-haltige Guanidine. Diese Guanidine werden bei der Aushärtung der Zusammensetzung kovalent angebunden, was Migrationseffekte weitgehend ausschliesst.

Das Silangruppen-haltige organische Polymer ist bevorzugt ein Polyolefin, Polyester, Polyamid, Poly(meth)acrylat oder Polyether oder eine Mischform dieser Polymere, welches jeweils eine oder bevorzugt mehrere Silangruppen trägt. Die Silangruppen können seitlich in der Kette oder endständig stehen und sind über ein C-Atom an das organische Polymer gebunden.

Besonders bevorzugt ist das Silangruppen-haltige organische Polymer ein Silangruppen-haltiges Polyolefin oder ein Silangruppen-haltiger Polyester oder ein Silangruppen-haltiges Poly(meth)acrylat oder ein Silangruppen-haltiger Polyether oder eine Mischform dieser Polymere. Am meisten bevorzugt ist ein Silangruppen-haltiger Polyether.

Als Silangruppen am organischen Polymer bevorzugt sind Endgruppen der Formel (IV), 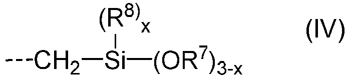 wobei
R⁷ für einen linearen oder verzweigten, einwertigen Kohlenwasserstoffrest mit 1 bis 5 C-Atomen, insbesondere für Methyl oder für Ethyl oder für Isopropyl, steht;
R⁸ für einen linearen oder verzweigten, einwertigen Kohlenwasserstoffrest mit 1 bis 8 C-Atomen, insbesondere für Methyl oder für Ethyl, steht; und x für einen Wert von 0 oder 1 oder 2, bevorzugt für 0 oder 1, insbesondere für 0, steht.
Besonders bevorzugt steht R⁷ für Methyl oder für Ethyl.

Für bestimmte Anwendungen steht der Rest R⁷ bevorzugt für eine Ethylgruppe, da in diesem Fall bei der Aushärtung der Zusammensetzung ökologisch und toxikologisch harmloses Ethanol freigesetzt wird.
Besonders bevorzugt sind Trimethoxysilangruppen, Dimethoxymethylsilangruppen oder Triethoxysilangruppen.
Dabei weisen Methoxysilangruppen den Vorteil auf, dass sie besonders reaktiv sind, und Ethoxysilangruppen weisen den Vorteil auf, dass sie toxikologisch vorteilhaft und besonders lagerstabil sind.

Das Silangruppen-haltige organische Polymer weist im Mittel bevorzugt 1.3 bis 4, insbesondere 1.5 bis 3, besonders bevorzugt 1.7 bis 2.8 Silangruppen pro Molekül auf. Die Silangruppen sind bevorzugt endständig.
Das Silangruppen-haltige organische Polymer weist bevorzugt ein mittleres Molekulargewicht, bestimmt mittels GPC gegenüber Polystyrol-Standard, im Bereich von 1000 bis 30'000 g/mol, insbesondere von 2000 bis 20'000 g/mol, auf. Das Silangruppen-haltige organische Polymer weist bevorzugt ein Silan-Equivalentgewicht von 300 bis 25'000 g/Eq, insbesondere von 500 bis 15'000 g/Eq, auf.

Das Silangruppen-haltige organische Polymer kann bei Raumtemperatur fest oder flüssig vorliegen. Bevorzugt ist es bei Raumtemperatur flüssig.

Meist bevorzugt handelt es sich beim Silangruppen-haltigen organischen Polymer um einen bei Raumtemperatur flüssigen Silangruppen-haltigen Polyether, wobei die Silangruppen insbesondere Dialkoxysilangruppen und/oder Trialkoxysilangruppen, besonders bevorzugt Trimethoxysilangruppen oder Triethoxysilangruppen, sind.

Verfahren zur Herstellung von Silangruppen-haltigen Polyethern sind dem Fachmann bekannt.
In einem Verfahren sind Silangruppen-haltige Polyether erhältlich aus der Umsetzung von Allylgruppen-haltigen Polyethern mit Hydrosilanen, gegebenenfalls unter Kettenverlängerung mit beispielsweise Diisocyanaten.

In einem weiteren Verfahren sind Silangruppen-haltige Polyether erhältlich aus der Copolymerisation von Alkylenoxiden und Epoxysilanen, gegebenenfalls unter Kettenverlängerung mit beispielsweise Diisocyanaten.
In einem weiteren Verfahren sind Silangruppen-haltige Polyether erhältlich aus der Umsetzung von Polyetherpolyolen mit Isocyanatosilanen, gegebenenfalls unter Kettenverlängerung mit Diisocyanaten.
In einem weiteren Verfahren sind Silangruppen-haltige Polyether erhältlich aus der Umsetzung von Isocyanatgruppen-haltigen Polyethern, insbesondere NCO-terminierten Urethan-Polyethern aus der Umsetzung von Polyetherpolyolen mit einer überstöchiometischen Menge an Polyisocyanaten, mit Aminosilanen, Hydroxysilanen oder Mercaptosilanen. Silangruppen-haltige Polyether aus diesem Verfahren sind besonders bevorzugt. Dieses Verfahren ermöglicht den Einsatz einer Vielzahl von kommerziell gut verfügbaren, kostengünstigen Ausgangsmaterialien, womit unterschiedliche Polymereigenschaften erhalten werden können, beispielsweise eine hohe Dehnbarkeit, eine hohe Festigkeit, ein niedriges Elastizitätsmodul, ein tiefer Glasübergangspunkt oder eine hohe Witterungsbeständigkeit.

Besonders bevorzugt ist der Silangruppen-haltige Polyether erhältlich aus der Umsetzung von NCO-terminierten Urethan-Polyethern mit Aminosilanen oder Hydroxysilanen. Geeignete NCO-terminierte Urethan-Polyether sind erhältlich aus der Umsetzung von Polyetherpolyolen, insbesondere Polyoxyalkylendiolen oder Polyoxyalkylentriolen, bevorzugt Polyoxypropylendiolen oder Polyoxypropylentriolen, mit einer überstöchiometrischen Menge an Polyisocyanaten, insbesondere Diisocyanaten.
Bevorzugt wird die Umsetzung zwischen dem Polyisocyanat und dem Polyetherpolyol unter Feuchtigkeitsausschluss bei einer Temperatur von 50 °C bis 160 °C durchgeführt, gegebenenfalls in Anwesenheit geeigneter Katalysatoren, wobei das Polyisocyanat so dosiert ist, dass dessen Isocyanatgruppen im Verhältnis zu den Hydroxylgruppen des Polyols im stöchiometrischen Überschuss vorhanden sind. Insbesondere wird der Überschuss an Polyisocyanat so gewählt, dass im resultierenden Urethan-Polyether nach der Umsetzung aller Hydroxylgruppen ein Gehalt an freien Isocyanatgruppen von 0.1 bis 5 Gewichts-%, bevorzugt 0.2 bis 4 Gewichts-%, besonders bevorzugt 0.3 bis 3 Gewichts-%, bezogen auf das gesamte Polymer, verbleibt.
Bevorzugte Diisocyanate sind ausgewählt aus der Gruppe bestehend aus 1,6-Hexamethylendiisocyanat (HDI), 1-Isocyanato-3,3,5-trimethyl-5-isocyanatomethyl-cyclohexan (=Isophorondiisocyanat oder IPDI), 2,4- und 2,6-Toluylendiisocyanat und beliebige Gemische dieser Isomeren (TDI) und 4,4'-, 2,4'- und 2,2'-Diphenylmethandiisocyanat und beliebige Gemische dieser Isomeren (MDI). Besonders bevorzugt sind IPDI oder TDI. Meist bevorzugt ist IPDI. Damit werden Silangruppen-haltige Polyether mit besonders guter Lichtechtheit erhalten.

Speziell geeignet als Polyetherpolyole sind Polyoxyalkylendiole oder Polyoxyalkylentriole mit einem Ungesättigtheitsgrad tiefer als 0.02 mEq/g, insbesondere tiefer als 0.01 mEq/g, und einem mittleren Molekulargewicht im Bereich von 400 bis 25'000 g/mol, insbesondere 1000 bis 20'000 g/mol.
Neben Polyetherpolyolen können anteilig auch andere Polyole eingesetzt werden, insbesondere Polyacrylatpolyole, sowie niedrigmolekulare Diole oder Triole.

Geeignete Aminosilane für die Umsetzung mit einem NCO-terminierten Urethan-Polyether sind primäre und sekundäre Aminosilane. Bevorzugt sind 3-Aminopropyltrimethoxysilan, 3-Aminopropyldimethoxymethylsilan, 4-Aminobutyl-trimethoxysilan, 4-Amino-3-methylbutyl-trimethoxysilan, 4-Amino-3,3-dimethylbutyl-trimethoxysilan, N-Butyl-3-aminopropyltrimethoxysilan, N-Phenyl-3-aminopropyltrimethoxysilan, Addukte aus primären Aminosilanen wie 3-Aminopropyltrimethoxysilan, 3-Aminopropyldimethoxymethylsilan oder N-(2-Aminoethyl)-3-aminopropyltrimethoxysilan und Michael-Akzeptoren wie Acrylnitril, (Meth)acrylsäureestern, (Meth)acrylsäureamiden, Maleinsäure- oder Fumarsäurediestern, Citraconsäurediestern oder Itaconsäurediestern, insbesondere N-(3-Trimethoxysilylpropyl)amino-bernsteinsäuredimethyl- oder -diethylester. Ebenfalls geeignet sind Analoga der genannten Aminosilane mit Ethoxy- oder Isopropoxygruppen anstelle der Methoxygruppen am Silicium. Geeignete Hydroxysilane für die Umsetzung mit einem NCO-terminierten Urethan-Polyether sind insbesondere erhältlich aus der Addition von Aminosilanen an Lactone oder an cyclische Carbonate oder an Lactide.
Dafür geeignete Aminosilane sind insbesondere 3-Aminopropyl-trimethoxysilan, 3-Aminopropyl-triethoxysilan, 4-Aminobutyl-trimethoxysilan, 4-Aminobutyl-triethoxysilan, 4-Amino-3-methylbutyl-trimethoxysilan, 4-Amino-3-methylbutyl-triethoxysilan, 4-Amino-3,3-dimethylbutyl-trimethoxysilan, 4-Amino-3,3-dimethylbutyl-triethoxysilan, 2-Aminoethyl-trimethoxysilan oder 2-Aminoethyltriethoxysilan. Besonders bevorzugt sind 3-Aminopropyl-trimethoxysilan, 3-Aminopropyl-triethoxysilan, 4-Amino-3,3-dimethylbutyl-trimethoxysilan oder 4-Amino-3,3-dimethylbutyl-triethoxysilan.
Als Lactone geeignet sind insbesondere γ-Valerolacton, γ-Octalacton, δ-Decalacton, und ε-Decalacton, insbesondere γ-Valerolacton.
Als cyclische Carbonate eignen sich insbesondere 4,5-Dimethyl-1,3-dioxolan-2-on, 4,4-Dimethyl-1,3-dioxolan-2-on, 4-Ethyl-1,3-dioxolan-2-on, 4-Methyl-1,3-dioxolan-2-on oder 4-(Phenoxymethyl)-1,3-dioxolan-2-on.
Als Lactide eignen sich insbesondere 1,4-Dioxan-2,5-dion (Lactid aus 2-Hydroxyessigsäure, auch "Glycolid" genannt), 3,6-Dimethyl-1,4-dioxan-2,5-dion (Lactid aus Milchsäure, auch "Lactid" genannt) und 3,6-Diphenyl-1,4-dioxan-2,5-dion (Lactid aus Mandelsäure).
Bevorzugte Hydroxysilane, welche auf diese Weise erhalten werden, sind N-(3-Triethoxysilylpropyl)-2-hydroxypropanamid, N-(3-Trimethoxysilylpropyl)-2-hydroxypropanamid, N-(3-Triethoxysilylpropyl)-4-hydroxypentanamid, N-(3-Triethoxysilylpropyl)-4-hydroxyoctanamid, N-(3-Triethoxysilylpropyl)-5-hydroxydecanamid und N-(3-Triethoxysilylpropyl)-2-hydroxypropylcarbamat.

Weiterhin sind geeignete Hydroxysilane auch erhältlich aus der Addition von Aminosilanen an Epoxide oder aus der Addition von Aminen an Epoxysilane. Bevorzugte Hydroxysilane, welche auf diese Weise erhalten werden, sind 2-Morpholino-4(5)-(2-trimethoxysilylethyl)cyclohexan-1-ol, 2-Morpholino-4(5)-(2-triethoxysilylethyl)cyclohexan-1-ol oder 1-Morpholino-3-(3-(triethoxysilyl)propoxy)propan-2-ol.

Als Silangruppen-haltige Polyether sind auch kommerziell erhältliche Produkte geeignet, insbesondere die Folgenden: MS Polymer™ (von Kaneka Corp.; insbesondere die Typen S203H, S303H, S227, S810, MA903 und S943); MS Polymer™ bzw. Silyl™ (von Kaneka Corp.; insbesondere die Typen SAT010, SAT030, SAT200, SAX350, SAX400, SAX725, MAX450, MAX951); Excestar® (von Asahi Glass Co. Ltd.; insbesondere die Typen S2410, S2420, S3430, S3630); SPUR+* (von Momentive Performance Materials; insbesondere die Typen 1010LM, 1015LM, 1050MM); Vorasil™ (von Dow Chemical Co.; insbesondere die Typen 602 und 604); Desmoseal® (von Bayer MaterialScience AG; insbesondere die Typen S XP 2458, S XP 2636, S XP 2749, S XP 2774 und S XP 2821), TEGOPAC® (von Evonik Industries AG; insbesondere die Typen Seal 100, Bond 150, Bond 250), Polymer ST (von Hanse Chemie AG/Evonik Industries AG, insbesondere die Typen 47, 48, 61, 61LV, 77, 80, 81); Geniosil® STP (von Wacker Chemie AG; insbesondere die Typen E10, E15, E30, E35).

Besonders bevorzugte Endgruppen der Formel (IV) sind Endgruppen der Formel (V), 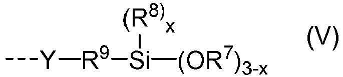 wobei
R⁹ für einen linearen oder verzweigten, zweiwertigen Kohlenwasserstoffrest mit 1 bis 12 C-Atomen, welcher gegebenenfalls cyclische und/oder aromatische Anteile und gegebenenfalls ein oder mehrere Heteroatome, insbesondere ein oder mehrere Stickstoffatome, aufweist, steht;
Y für einen zweiwertigen Rest ausgewählt aus -O-, -S-, -N(R¹⁰)-, -O-CO-N(R¹⁰)-, -N(R¹⁰)-CO-O- und -N(R¹⁰)-CO-N(R¹⁰)- steht,
   wobei R¹⁰ für Wasserstoff oder für einen linearen oder verzweigten Kohlenwasserstoff-Rest mit 1 bis 20 C-Atomen, welcher gegebenenfalls cyclische Anteile aufweist, und welcher gegebenenfalls eine Alkoxysilyl-, Ether- oder Carbonsäureestergruppe aufweist, steht; und
R⁷, R⁸ und x die bereits genannten Bedeutungen aufweisen.
Bevorzugt steht R⁹ für 1,3-Propylen oder für 1,4-Butylen, wobei Butylen mit einer oder zwei Methylgruppen substituiert sein kann.

Besonders bevorzugt steht R⁹ für 1,3-Propylen.

Zusätzlich zum Katalysator der Formel (I) kann die erfindungsgemässe Zusammensetzung weitere Katalysatoren für die Vernetzung der Silangruppen enthalten. Als weitere Katalysatoren geeignet sind insbesondere Metall-Katalysatoren und/oder basische Stickstoff- oder Phosphorverbindungen.

Mögliche Metall-Katalysatoren sind insbesondere Verbindungen von Zinn, Titan, Zirkonium, Aluminium oder Zink, insbesondere Diorganozinn(IV)-Verbindungen wie insbesondere Dibutylzinn(IV)-diacetat, Dibutylzinn(IV)-dilaurat, Dibutylzinn(IV)-dineodecanoat oder Dibutylzinn(IV)-bis(acetylacetonat) und Dioctylzinn(IV)-dilaurat, sowie Titan(IV)- oder Zirkonium(IV)- oder Aluminium(III)- oder Zink(II)-Komplexe mit insbesondere Alkoxy-, Carboxylat-, 1,3-Diketonat-, 1,3-Ketoesterat- oder 1,3-Ketoamidat-Liganden.

Mögliche basische Stickstoff- oder Phosphorverbindungen sind insbesondere Imidazole, Pyridine, Phosphazen-Basen oder bevorzugt Amine, Hexahydrotriazine, Biguanide, Amidine oder Guanidine, welche nicht der Formel (I) entsprechen.

Geeignete Amine sind insbesondere Alkyl-, Cycloalkyl- oder Aralkylamine wie Triethylamin, Triisopropylamin, 1-Butylamin, 2-Butylamin, tert.Butylamin, 3-Methyl-1-butylamin, 3-Methyl-2-butylamin, Dibutylamin, Tributylamin, Hexylamin, Dihexylamin, Cyclohexylamin, Dicyclohexylamin, Dimethylcyclohexylamin, Benzylamin, Dibenzylamin, Dimethylbenzylamin, Octylamin, 2-Ethylhexylamin, Di-(2-ethylhexyl)amin, Laurylamin, N,N-Dimethyl-laurylamin, Stearylamin, N,N-Dimethyl-stearylamin; von natürlichen Fettsäuregemischen abgeleitete Fettamine, wie insbesondere Cocoalkylamin, N,N-Dimethyl-cocoalkylamin, C₁₆₋₂₂-Alkylamin, N,N-Dimethyl-C₁₆₋₂₂-alkylamin, Soyaalkylamin, N,N-Dimethyl-soyaalkylamin, Oleylamin, N,N-Dimethyl-oleylamin, Talgalkylamin oder N,N-Dimethyl-talgalkylamin, erhältlich beispielsweise unter den Handelsnamen Armeen® (von Akzo Nobel) oder Rofamin® (von Ecogreen Oleochemicals); aliphatische, cycloaliphatische oder araliphatische Diamine wie Ethylendiamin, Butandiamin, Hexamethylendiamin, Dodecandiamin, Neopentandiamin, 2-Methyl-pentamethylendiamin (MPMD), 2,2(4),4-Trimethylhexamethylendiamin (TMD), Isophorondiamin (IPD), 2,5(2,6)-Bis-(aminomethyl)-bicyclo[2.2.1]heptan (NBDA), 1,3-Xylylendiamin (MXDA), N,N'-Di(tert.butyl)ethylendiamin, N,N,N',N'-Tetramethyl-ethylendiamin, N,N,N',N'-Tetramethyl-propylendiamin, N,N,N',N'-Tetramethyl-hexamethylendiamin, 3-Dimethylaminopropylamin, 3-(Methylamino)propylamin, 3-(Cyclohexylamino)propylamin, Piperazin, N-Methylpiperazin, N,N'-Dimethylpiperazin, 1,4-Diazabicyclo[2.2.2]octan, Fettpolyamine wie N-Cocoalkyl-1,3-propandiamin, N-Oleyl-1,3-propandiamin, N-Soyaalkyl-1,3-propandiamin, N-Talgalkyl-1,3-propandiamin oder N-(C₁₆₋₂₂-Al-kyl)-1,3-propandiamin, erhältlich beispielsweise unter dem Handelsnamen Duomeen® (von Akzo Nobel); Polyalkylenamine wie Diethylentriamin, Dipropylentriamin, Triethylentetramin (TETA), Tetraethylenpentamin (TEPA), Pentamethylenhexamin (PEHA), 3-(2-Aminoethyl)aminopropylamin, N,N'-Bis(3-aminopropyl)ethylendiamin, N-(3-Aminopropyl)-N-methylpropandiamin, Bis(3-dimethylaminopropyl)amin, N-(3-Dimethylaminopropyl)-1,3-propylendiamin, N-(2-Aminoethyl)piperazin (N-AEP), N-(2-Aminopropyl)piperazin, N,N'-Di-(2-aminoethyl)piperazin, 1-Methyl-4-(2-dimethylaminoethyl)piperazin, N,N,N',N",N"-Pentamethyldiethylentriamin, N,N,N',N",N"-Pentamethyldipropylentriamin, Polyethylenimine, erhältlich beispielsweise unter den Handelsnamen Lupasol® (von BASF) und Epomin® (von Nippon Shokubai); Etheramine, wie insbesondere 2-Methoxyethylamin, 2-Ethoxyethylamin, 3-Methoxypropylamin, 3-Ethoxypropylamin, 3-(2-Ethylhexyloxy)propylamin, 3-(2-Methoxyethoxy)propylamin, 2(4)-Methoxyphenylethylamin, Morpholin, N-Methylmorpholin, N-Ethylmorpholin, 2-Aminoethylmorpholin, Bis(2-aminoethyl)ether, Bis(dimethylaminoethyl)-ether, Bis(dimorpholinoethyl)ether, N,N,N'-Trimethyl-N'-hydroxyethylbis(2-aminoethyl)ether, 3,6-Dioxaoctan-1,8-diamin, 4,7-Dioxadecan-1,10-diamin, 4,7-Dioxadecan-2,9-diamin, 4,9-Dioxadodecan-1,12-diamin, 5,8-Dioxadodecan-3,10-diamin, 4,7,10-Trioxatridecan-1,13-diamin oder 2-Aminopropyl-terminierte Glykole, wie sie beispielsweise unter dem Handelsnamen Jeffamin® (von Huntsman) erhältlich sind; Aminoalkohole, wie insbesondere Ethanolamin, Isopropanolamin, Diethanolamin, Diisopopanolamin, Triethanolamin, Triisopropanolamin, N-Butylethanolamin, Diglykolamin, N,N-Diethylethanolamin, N-Methyldiethanolamin, N-Methyldiisopropylamin, N,N,N'-Trimethylaminoethyl-ethanolamin, N-(3-Dimethylaminopropyl)-N,N-diisopropanolamin, N,N-Bis(3-dimethylaminopropyl)-N-isopropanolamin, 2-(2-Dimethylaminoethoxy)ethanolamin oder Addukte aus Mono- und Polyaminen mit Epoxiden oder Diepoxiden; Phenolgruppen-haltige Amine, wie insbesondere Kondensationsprodukte aus Phenolen, Aldehyden und Aminen (sogenannte Mannich-Basen und Phenalkamine) wie insbesondere 2-(Dimethylaminomethyl)phenol, 2,4,6-Tris(dimethylaminomethyl)phenol oder Polymere aus Phenol, Formaldehyd und N,N-Dimethyl-1,3-propandiamin sowie im Handel unter den Markennamen Cardolite® (von Cardolite), Aradur® (von Huntsman) und Beckopox® (von Cytec) erhältliche Phenalkamine; Amidgruppen-haltige Polyamine, sogenannte Polyamidoamine, wie sie im Handel erhältlich sind, beispielsweise unter den Markennamen Versamid® (von Cognis), Aradur® (von Huntsman), Euretek® (von Huntsman) oder Beckopox® (von Cytec); oder Aminosilane, wie insbesondere 3-Aminopropyltrimethoxysilan, 3-Aminopropyldimethoxymethylsilan, N-(2-Aminoethyl)-3-aminopropyltrimethoxysilan, N-(2-Aminoethyl)-3-aminopropyl-methyldimethoxysilan, N-(2-Aminoethyl)-N'-[3-(trimethoxysilyl)propyl]ethylendiamin oder deren Analoga mit Ethoxy- anstelle der Methoxygruppen am Silicium.
Geeignete Triazine sind insbesondere 1,3,5-Hexahydrotriazin oder 1,3,5-Tris(3-(dimethylamino)propyl)-hexahydrotriazin.
Geeignete Biguanide sind insbesondere Biguanid, 1-Butylbiguanid, 1,1-Dimethylbiguanid, 1-Butylbiguanid, 1-Phenylbiguanid oder 1-(o-Tolyl)biguanid (OTBG).
Geeignete Amidine sind insbesondere 1,8-Diazabicyclo[5.4.0]undec-7-en (DBU), 1,5-Diazabicyclo[4.3.0]non-5-en (DBN), 6-Dibutylamino-1,8-diazabicyclo[5.4.0]undec-7-en, 6-Dibutylamino-1,8-diazabicyclo[5.4.0]undec-7-en, N,N'-Di-n-hexylacetamidin (DHA), 2-Methyl-1,4,5,6-tetrahydropyrimidin, 1,2-Dimethyl-1,4,5,6-tetrahydropyrimidin, 2,5,5-Trimethyl-1,4,5,6-tetrahydropyrimidin, N-(3-Trimethoxysilylpropyl)-4,5-dihydroimidazol oder N-(3-Triethoxysilylpropyl)-4,5-dihydroimidazol.

Geeignete Guanidine, welche nicht der Formel (I) entsprechen, sind insbesondere 1-Butylguanidin, 1,1-Dimethylguanidin, 1,3-Dimethylguanidin, 1,1,3,3-Tetramethylguanidin (TMG), 2-(3-(Trimethoxysilyl)propyl)-1,1,3,3-tetramethylguanidin, 2-(3-(Methyldimethoxysilyl)propyl)-1,1,3,3-tetramethylguanidin, 2-(3-(Triethoxysilyl)propyl)-1,1,3,3-tetramethylguanidin, 1,5,7-Triazabicyclo[4.4.0]-dec-5-en (TBD), 7-Methyl-1,5,7-triazabicyclo[4.4.0]dec-5-en, 7-Cyclohexyl-1,5,7-triazabicyclo[4.4.0]dec-5-en, 1-Phenylguanidin, 1-(o-Tolyl)guanidin (OTG), 1,3-Diphenylguanidin, 1,3-Di(o-tolyl)guanidin oder 2-Guanidinobenzimidazol.

Weiterhin kann die erfindungsgemässe Zusammensetzung als Co-Katalysator eine Säure enthalten, insbesondere eine Carbonsäure. Bevorzugt sind aliphatische Carbonsäuren wie Ameisensäure, Laurinsäure, Stearinsäure, Isostearinsäure, Ölsäure, 2-Ethyl-2,5-dimethylcapronsäure, 2-Ethylhexansäure, Neodecansäure, Fettsäuregemische aus der Verseifung von natürlichen Fetten und Ölen oder Di- und Polycarbonsäuren, insbesondere Poly(meth)acrylsäuren.

Die erfindungsgemässe Zusammensetzung ist in einer bevorzugten Ausführungsform im Wesentlichen frei von Organozinn-Verbindungen. Organozinnfreie Zusammensetzungen sind hinsichtlich Gesundheitsschutz und Umweltschutz vorteilhaft. Insbesondere beträgt der Zinngehalt der Zusammensetzung weniger als 0.1 Gew.-%, insbesondere weniger als 0.05 Gew.-%.

In einer weiteren bevorzugten Ausführungsform enthält die erfindungsgemässe Zusammensetzung eine Kombination aus mindestens einem Katalysator der Formel (I) und mindestens einer Organozinn-Verbindung, insbesondere einer Diorganozinn(IV)-Verbindung wie die vorgängig genannten. Eine solche Zusammensetzung weist bereits bei einem geringen Zinn-Gehalt eine hohe Aushärtungsgeschwindigkeit auf, was aus toxikologischen und ökologischen Gründen vorteilhaft ist. Der Zinn-Gehalt der Zusammensetzung beträgt dabei insbesondere weniger als 1 Gewichts-%.

In einer Ausführungsform der Erfindung enthält die Zusammensetzung neben dem Silangruppen-haltigen organischen Polymer und einem Katalysator der Formel (I) zusätzlich mindestens ein Organotitanat. Eine Kombination aus Katalysator der Formel (I) und Organotitanat weist eine besonders hohe katalytische Aktivität auf. Dadurch wird eine schnelle Aushärtung der Zusammensetzung bei verhältnismässig geringer Einsatzmenge des Katalysators der Formel (I) ermöglicht.
Als Organotitanat geeignet sind insbesondere Titan(IV)-Komplexverbindungen. Bevorzugte Organoititanate sind insbesondere ausgewählt aus
- Titan(IV)-Komplexverbindungen mit zwei 1,3-Diketonat-Liganden, insbesondere 2,4-Pentandionat (=Acetylacetonat), und zwei Alkoholat-Liganden;
- Titan(IV)-Komplexverbindungen mit zwei 1,3-Ketoesterat-Liganden, insbesondere Ethylacetoacetat, und zwei Alkoholat-Liganden;
- Titan(IV)-Komplexverbindungen mit einem oder mehreren Aminoalkoholat-Liganden, insbesondere Triethanolamin oder 2-((2-Aminoethyl)amino)ethanol, und einem oder mehreren Alkoholat-Liganden;
- Titan(IV)-Komplexverbindungen mit vier Alkoholat-Liganden;
- sowie höherkondensierte Organotitanate, insbesondere oligomeres Titan(IV)-tetrabutanolat, auch als Polybutyltitanat bezeichnet;
wobei als Alkoholat-Liganden insbesondere Isobutoxy, n-Butoxy, Isopropoxy, Ethoxy und 2-Ethylhexoxy geeignet sind.

Insbesondere geeignet sind die kommerziell erhältlichen Typen Tyzor® AA, GBA, GBO, AA-75, AA-65, AA-105, DC, BEAT, BTP, TE, TnBT, KTM, TOT, TPT oder IBAY (alle von Dorf Ketal); Tytan PBT, TET, X85, TAA, ET, S2, S4 oder S6 (alle von Borica Company Ltd.) und Ken-React® KR® TTS, 7, 9QS, 12, 26S, 33DS, 38S, 39DS, 44, 134S, 138S, 133DS, 158FS oder LICA® 44 (alle von Kenrich Petrochemicals).

Ganz besonders geeignete Organotitanate sind ausgewählt aus Bis(ethylacetoacetato)diisobutoxy-titan(IV) (kommerziell erhältlich beispielsweise als Tyzor® IBAY von Dorf Ketal), Bis(ethylacetoacetato)diisopropoxy-titan(IV) (kommerziell erhältlich beispielsweise als Tyzor® DC von Dorf Ketal), Bis(acetylacetonato)diisopropoxy-titan(IV), Bis(acetylacetonato)diisobutoxy-titan(IV), Tris(oxyethyl)-amin-isopropoxy-titan(IV), Bis[tris(oxyethyl)amin]diisopropoxy-titan(IV), Bis(2-ethylhexan-1,3-dioxy)-titan(IV), Tris[2-((2-Aminoethyl)amino)ethoxy]ethoxy-titan(IV), Bis(neopentyl(diallyl)oxy)-diethoxy-titan(IV), Titan(IV)-tetrabutanolat, Tetra(2-ethylhexyloxy)titanat, Tetra(isopropoxy)titanat und Polybutyltitanat. Am meisten bevorzugt sind Bis(ethylacetoacetato)diisobutoxy-titan(IV) oder Bis(ethylacetoacetato)diisopropoxy-titan(IV).

In einer Organotitanat-haltigen Zusammensetzung liegt das Gewichtsverhältnis zwischen dem Silangruppen-haltigen organischen Polymer und dem Katalysator der Formel (I) bevorzugt im Bereich von 40/1 bis 2'000/1.
Das Gewichtsverhältnis zwischen dem Organotitanat und dem Katalysator der Formel (I) liegt bevorzugt im Bereich von 10/1 bis 1/10, besonders bevorzugt 5/1 bis 1/5, insbesondere 5/1 bis 1/3.

Die erfindungsgemässe Zusammensetzung kann weitere Bestandteile enthalten, insbesondere die folgenden Hilfs- und Zusatzmittel:
- Haftvermittler und/oder Vernetzer, insbesondere Aminosilane wie insbesondere 3-Aminopropyl-trimethoxysilan, 3-Aminopropyl-dimethoxymethylsilan, N-(2-Aminoethyl)-3-aminopropyl-trimethoxysilan, N-(2-Aminoethyl)-3-aminopropyl-dimethoxymethylsilan, N-(2-Aminoethyl)-N'-[3-(trimethoxysilyl)propyl]-ethylendiamin oder deren Analoga mit Ethoxy- anstelle von Methoxygruppen, weiterhin N-Phenyl-, N-Cyclohexyl- oder N-Alkylaminosilane, Mercaptosilane, Epoxysilane, (Meth)acrylosilane, Anhydridosilane, Carbamatosilane, Alkylsilane oder Iminosilane, oligomere Formen dieser Silane, Addukte aus primären Aminosilanen mit Epoxysilanen oder (Meth)acrylosilanen oder Anhydridosilanen. Insbesondere geeignet sind 3-Aminopropyltrimethoxysilan, 3-Aminopropyltriethoxysilan, N-(2-Aminoethyl)-3-aminopropyltrimethoxysilan, N-(2-Aminoethyl)-3-aminopropyl-triethoxysilan, 3-Glycidoxypropyltrimethoxysilan, 3-Glycidoxypropyltriethoxysilan oder 3-Ureidopropyltrimethoxysilan, oder oligomere Formen dieser Silane;
- Trocknungsmittel, insbesondere Tetraethoxysilan, Vinyltrimethoxysilan, Vinyltriethoxysilan oder Organoalkoxysilane, welche in α-Stellung zur Silangruppe eine funktionelle Gruppe aufweisen, insbesondere N-(Methyldime-thoxysilylmethyl)-O-methyl-carbamat, (Methacryloxymethyl)silane, Methoxymethylsilane, Orthoameisensäureester, Calciumoxid oder Molekularsiebe, insbesondere Vinyltrimethoxysilan oder Vinyltriethoxysilan;
- Weichmacher, insbesondere Carbonsäureester wie Phthalate, insbesondere Dioctylphthalat, Bis(2-ethylhexyl)phthalat, Bis(3-propylheptyl)phthalat, Diisononylphthalat oder Diisodecylphthalat, Diester von ortho-Cyclohexandicarbonsäure, insbesondere Diisononyl-1,2-cyclohexandicarboxylat, Adipate, insbesondere Dioctyladipat, Bis(2-Ethylhexyl)adipat, Azelate, insbesondere Bis(2-ethylhexyl)acelat, Sebacate, insbesondere Bis(2-ethylhexyl)sebacat oder Diisononylsebacat, Polyole, insbesondere Polyoxyalkylenpolyole oder Polyesterpolyole, Glykolether, Glykolester, organische Phosphor- oder Sulfonsäureester, Sulfonsäureamide, Polybutene oder von natürlichen Fetten oder Ölen abgeleitete Fettsäuremethyl- oder -ethylester, auch "Biodiesel" genannt;
- Lösemittel;
- anorganische oder organische Füllstoffe, insbesondere natürliche, gemahlene oder gefällte Calciumcarbonate, welche gegebenenfalls mit Fettsäuren, insbesondere Stearinsäure, beschichtet sind, Baryt (Schwerspat), Talke, Quarzmehle, Quarzsand, Dolomite, Wollastonite, Kaoline, calcinierte Kaoline, Mica (Kalium-Aluminium-Silikat), Molekularsiebe, Aluminiumoxide, Aluminiumhydroxide, Magnesiumhydroxid, Kieselsäuren inklusive hochdisperse Kieselsäuren aus Pyrolyseprozessen, industriell hergestellte Russe, Graphit, Metall-Pulver wie Aluminium, Kupfer, Eisen, Silber oder Stahl, PVC-Pulver oder Hohlkugeln;
- Fasern, insbesondere Glasfasern, Kohlefasern, Metallfasern, Keramikfasern oder Kunststofffasern wie Polyamidfasern oder Polyethylenfasern;
- Farbstoffe;
- Pigmente, insbesondere Titandioxid oder Eisenoxide;
- Rheologie-Modifizierer, insbesondere Verdickungsmittel, insbesondere Schichtsilikate wie Bentonite, Derivate von Rizinusöl, hydriertes Rizinusöl, Polyamide, Polyurethane, Harnstoffverbindungen, pyrogene Kieselsäuren, Celluloseether oder hydrophob modifizierte Polyoxyethylene;
- Stabilisatoren gegen Oxidation, Wärme, Licht und UV-Strahlung;
- natürliche Harze, Fette oder Öle wie Kolophonium, Schellack, Leinöl, Rizinusöl oder Sojaöl;
- nicht-reaktive Polymere, wie insbesondere Homo- oder Copolymere von ungesättigten Monomeren, insbesondere aus der Gruppe umfassend Ethylen, Propylen, Butylen, Isobutylen, Isopren, Vinylacetat oder Alkyl(meth)acrylate, insbesondere Polyethylene (PE), Polypropylene (PP), Polyisobutylene, Ethylenvinylacetat-Copolymere (EVA) oder ataktische Poly-α-Olefine (APAO);
- flammhemmende Substanzen, insbesondere die bereits genannten Füllstoffe Aluminiumhydroxid und Magnesiumhydroxid, oder insbesondere organische Phosphorsäureester wie insbesondere Triethylphosphat, Trikresylphosphat, Triphenylphosphat, Diphenylkresylphosphat, Isodecyldiphenylphosphat, Tris(1,3-dichlor-2-propyl)phosphat, Tris(2-chlorethyl)phosphat, Tris(2-ethylhexyl)phosphat, Tris(chlorisopropyl)phosphat, Tris(chlorpropyl)-phosphat, isopropyliertes Triphenylphosphat, Mono-, Bis-oder Tris(isopropylphenyl)phosphate unterschiedlichen Isopropylierungsgrades, Resorcinol-bis(diphenylphosphat), Bisphenol-A-bis(diphenylphosphat) oder Ammoniumpolyphosphate;
- oberflächenaktive Substanzen, insbesondere Netzmittel, Verlaufsmittel, Entlüftungsmittel oder Entschäumer;
- Biozide, insbesondere Algizide, Fungizide oder das Pilzwachstum hemmende Substanzen;
sowie weitere üblicherweise in feuchtigkeitshärtenden Zusammensetzungen eingesetzte Substanzen.Es kann sinnvoll sein, gewisse Bestandteile vor dem Einmischen in die Zusammensetzung chemisch oder physikalisch zu trocknen.

In einer bevorzugten Ausführungsform enthält die Zusammensetzung mindestens ein Trocknungsmittel und mindestens einen Haftvermittler und/oder Vernetzer.

In einer bevorzugten Ausführungsform enthält die Zusammensetzung keine Phtalate als Weichmacher. Solche Zusammensetzungen sind toxikologisch vorteilhaft und weisen weniger Probleme mit Migrationseffekten auf.

Die erfindungsgemässe Zusammensetzung wird vorzugsweise unter Ausschluss von Feuchtigkeit hergestellt und aufbewahrt. Typischerweise ist die Zusammensetzung in einer geeigneten Verpackung oder Anordnung, wie insbesondere einer Flasche, einer Büchse, einem Beutel, einem Eimer, einem Fass oder einer Kartusche, unter Ausschluss von Feuchtigkeit lagerstabil.

Die erfindungsgemässe Zusammensetzung kann in Form einer einkomponentigen oder in Form einer mehrkomponentigen, insbesondere zweikomponentigen, Zusammensetzung vorliegen. Bevorzugt handelt es sich bei der erfindungsgemässen Zusammensetzung um eine einkomponentige Zusammensetzung.
Als "einkomponentig" wird im vorliegenden Dokument eine Zusammensetzung bezeichnet, bei welcher alle Bestandteile der Zusammensetzung vermischt im gleichen Gebinde gelagert werden und welche mit Feuchtigkeit härtbar ist.
Als "zweikomponentig" wird im vorliegenden Dokument eine Zusammensetzung bezeichnet, bei welcher die Bestandteile der Zusammensetzung in zwei verschiedenen Komponenten vorliegen, welche in voneinander getrennten Gebinden gelagert werden. Erst kurz vor oder während der Applikation der Zusammensetzung werden die beiden Komponenten miteinander vermischt, worauf die vermischte Zusammensetzung aushärtet, wobei die Aushärtung erst durch die Einwirkung von Feuchtigkeit abläuft oder vervollständigt wird.

Die erfindungsgemässe Zusammensetzung wird insbesondere in einem Temperaturbereich zwischen 5 und 45 °C, bevorzugt bei Umgebungstemperatur, appliziert und härtet auch bei diesen Bedingungen aus.

Bei der Applikation der beschriebenen Zusammensetzung auf mindestens einen Festkörper oder Artikel kommen die in der Zusammensetzung enthaltenen Silangruppen in Kontakt mit Feuchtigkeit. Bei Kontakt mit Feuchtigkeit hydrolysieren die Silangruppen. Dabei bilden sich Silanolgruppen (Si-OH-Gruppen) und durch nachfolgende Kondensationsreaktionen Siloxangruppen (Si-O-Si-Gruppen). Gegebenenfalls vorhandene weitere feuchtigkeitsreaktive Gruppen reagieren ebenfalls mit vorhandener Feuchtigkeit. Als Ergebnis dieser Reaktionen härtet die Zusammensetzung aus. Der Katalysator der Formel (I) beschleunigt diese Aushärtung.
Das für die Aushärtung benötigte Wasser kann entweder aus der Luft stammen (Luftfeuchtigkeit), oder aber die vorgängig beschriebene Zusammensetzung kann mit einer Wasser enthaltenden Komponente in Kontakt gebracht werden, zum Beispiel durch Bestreichen, beispielsweise mit einem Abglättmittel, oder durch Besprühen, oder es kann der Zusammensetzung bei der Applikation Wasser oder eine Wasser enthaltende Komponente zugesetzt werden, zum Beispiel in Form einer wasserhaltigen oder Wasser freisetzenden Flüssigkeit oder Paste. Eine Paste ist insbesondere geeignet für den Fall, dass die Zusammensetzung selber in Form einer Paste vorliegt. Sie wird insbesondere über einen Statikmischer oder über einen dynamischen Mischer eingemischt. Eine derartige Wasser enthaltende Komponente wird der erfindungsgemässen Zusammensetzung typischerweise in einem Gewichtsverhältnis von erfindungsgemässer Zusammensetzung zu Wasser enthaltenden Komponente im Bereich von 5:1 bis 200:1, bevorzugt 10:1 bis 100:1, insbesondere 10:1 bis 50:1, zugesetzt.
Bei der Aushärtung mittels Luftfeuchtigkeit härtet die Zusammensetzung von aussen nach innen durch, wobei zunächst eine Haut an der Oberfläche der Zusammensetzung gebildet wird. Die sogenannte Hautbildungszeit stellt ein Mass für die Aushärtungsgeschwindigkeit der Zusammensetzung dar.
Die Geschwindigkeit der Aushärtung wird dabei im Allgemeinen von verschiedenen Faktoren, wie beispielsweise der Verfügbarkeit von Wasser, der Temperatur usw., bestimmt.

Die erfindungsgemässe Zusammensetzung verfügt über eine gute Lagerfähigkeit, erlaubt aufgrund der geringen Toxizität und geringen Flüchtigkeit des Katalysators der Forrmel (I) eine niedrige Gefahreneinstufung und ermöglicht emissions- und geruchsarme Zusammensetzungen, welche rasch aushärten und dabei ein mechanisch hochwertiges und beständiges Material bilden. Besonders vorteilhaft ist dabei der Umstand, dass dieses Material kaum zu migrationsbedingten Fehlern wie Ausschwitzen oder Substratverschmutzung neigt, im Gegensatz zu Zusammensetzungen enthaltend Katalysatoren nach dem Stand der Technik, wie beispielsweise DBU, TMG, DHA oder Titan(IV)-Komplexe. Zusammensetzungen enthaltend solche Katalysatoren neigen nach der Aushärtung zu Migrationseffekten, was sich nach der Aushärtung an klebrigen und/oder schmierigen Oberflächen und/oder an Substratverschmutzungen zeigen kann. Solche Effekte sind äusserst unerwünscht, da klebrige und schmierige Oberflächen schnell verschmutzen und schlecht überstreichbar sind, und Substratverunreinigungen zu bleibenden Verfärbungen führen können.

Die erfindungsgemässe Zusammensetzung eignet sich für eine Vielzahl von Verwendungen, insbesondere als Anstrich, Lack oder Primer, als Harz zur Herstellung von Faserverbundwerkstoff (Composite), als Vergussmasse, Dichtstoff, Klebstoff, Belag, Beschichtung oder Anstrich für Bau- und Industrieanwendungen, beispielsweise als Nahtabdichtung, Hohlraumversiegelung, Elektroisolationsmasse, Spachtelmasse, Fugendichtstoff, Schweiss- oder Bördelnahtdichtstoff, , Montageklebstoff, Karrosserieklebstoff, Scheibenklebstoff, Sandwichelementklebstoff, Kaschierklebstoff, Laminatklebstoff, Verpackungsklebstoff, Holzklebstoff, Parkettklebstoff, Verankerungsklebstoff, Bodenbelag, Bodenbeschichtung, Balkonbeschichtung, Dachbeschichtung, Betonschutzbeschichtung, Parkhausbeschichtung, Versiegelung, Rohrbeschichtung, Korrosionsschutzbeschichtung, Textilbeschichtung, Dämpfungselement, Dichtungselement oder Spachtelmasse.

Bei der Applikation wird die erfindungsgemässe Zusammensetzung auf mindestens ein Substrat appliziert.
Geeignete Substrate sind insbesondere
- Glas, Glaskeramik, Beton, Mörtel, Backstein, Ziegel, Gips oder Natursteine wie Kalkstein, Granit oder Marmor;
- Metalle und Legierungen, wie Aluminium, Eisen, Stahl oder Buntmetalle, sowie oberflächenveredelte Metalle oder Legierungen, wie verzinkte oder verchromte Metalle;
- Leder, Textilien, Papier, Holz, mit Harzen, beispielsweise Phenol-, Melamin- oder Epoxidharzen, gebundene Holzwerkstoffe, Harz-Textil-Verbundwerkstoffe und weitere sogenannte Polymer-Composites;
- Kunststoffe, wie Polyvinylchlorid (Hart- und Weich-PVC), Acrylonitril-Butadien-Styrol-Copolymere (ABS), Polycarbonat (PC), Polyamid (PA), Polyester, Poly(methylmethacrylat) (PMMA), Epoxidharze, Polyurethane (PUR), Polyoxymethylen (POM), Polyolefine (PO), Polyethylen (PE) oder Polypropylen (PP), Ethylen/Propylen-Copolymere (EPM) oder Ethylen/Propylen/Dien-Terpolymere (EPDM), oder faserverstärkte Kunststoffe wie Kohlefaserverstärkte Kunststoffe (CFK), Glasfaser-verstärkte Kunststoffe (GFK) oder Sheet Moulding Compounds (SMC), wobei die Kunststoffe bevorzugt mittels Plasma, Corona oder Flammen oberflächenbehandelt sein können;
- beschichtete Substrate, wie pulverbeschichtete Metalle oder Legierungen;
- Farben oder Lacke, insbesondere Automobildecklacke.
Die Substrate können bei Bedarf vor dem Applizieren der Zusammensetzung vorbehandelt werden, insbesondere durch physikalische und/oder chemische Reinigungsverfahren oder durch das Aufbringen eines Haftvermittlers, einer Haftvermittlerlösung oder eines Primers.
Besonders geeignet ist die erfindungsgemässe Zusammensetzung für den Kontakt mit Substraten, die besonders empfindlich sind auf Störungen durch migrierende Substanzen, insbesondere durch das Ausbilden von Verfärbungen oder Flecken. Dies sind insbesondere feinporige Substrate wie Marmor, Kalkstein oder andere Natursteine, Gips, Zementmörtel oder Beton, aber auch Kunststoffe. Insbesondere auf PVC werden bei der Anwesenheit von Katalysatoren wie beispielsweise DBU oder TMG starke Verfärbungen beobachtet, die sich durch Reinigung nicht entfernen lassen. Solche Effekte werden mit den Katalysatoren der Formel (I) nicht beobachtet.

Besonders geeignet ist die Zusammensetzung als Klebstoff und/oder Dichtstoff, insbesondere für die Fugenabdichtung und für elastische Klebeverbindungen in Bau- und Industrieanwendungen, sowie als elastische Beschichtung mit rissüberbrückenden Eigenschaften, insbesondere zum Schützen und/oder Abdichten von beispielsweise Dächern, Böden, Balkonen, Parkdecks oder Betonröhren.

Insbesondere betrifft die vorliegende Erfindung somit auch die Verwendung einer vorhergehend beschriebenen Zusammensetzung als Klebstoff, Dichtstoff oder Beschichtung.
Eine solche Zusammensetzung enthält typischerweise Weichmacher, Füllstoffe, Haftvermittler und/oder Vernetzer und Trocknungsmittel und gegebenenfalls weitere Hilfs- und Zusatzstoffe.
Üblicherweise beträgt dabei der Anteil an Silangruppen-haltigem organischen Polymer in der erfindungsgemässen Zusammensetzung 10 bis 80 Gew.-%, insbesondere 15 bis 60 Gew.-%, bevorzugt 15 bis 50 Gew.-%, bezogen auf das Gesamtgewicht der Zusammensetzung.

Für eine Anwendung als Klebstoff oder Dichtstoff weist die Zusammensetzung bevorzugt eine pastöse Konsistenz mit strukturviskosen Eigenschaften auf. Ein solcher pastöser Dichtstoff oder Klebstoff wird insbesondere aus handelsüblichen Kartuschen, welche manuell, mittels Druckluft oder Batterie betrieben werden, oder aus einem Fass oder Hobbock mittels einer Förderpumpe oder eines Extruders, gegebenenfalls mittels eines Applikationsroboters, auf ein Substrat aufgetragen.
Für eine Anwendung als Beschichtung weist die Zusammensetzung bevorzugt eine bei Raumtemperatur flüssige Konsistenz mit selbstverlaufenden Eigenschaften auf. Gegebenenfalls ist sie leicht thixotrop, so dass die Beschichtung an abschüssigen bis senkrechten Flächen applizierbar ist, ohne sofort wegzufliessen. Sie wird insbesondere appliziert mittels Roller oder Pinsel oder durch Ausgiessen und Verteilen mittels beispielsweise einem Roller, einem Schaber oder einer Zahntraufel.

Verklebt oder abgedichtet werden können zwei gleichartige oder zwei verschiedene Substrate, insbesondere die vorgängig genannten Substrate.

Weiterhin betrifft die Erfindung eine ausgehärtete Zusammensetzung, welche erhältlich ist aus einer Zusammensetzung, wie sie vorhergehend beschrieben worden ist, nach deren Härtung mit Wasser, insbesondere in Form von Luftfeuchtigkeit.
Die ausgehärtete Zusammensetzung zeichnet sich dadurch aus, dass sie kaum oder gar nicht zu migrationsbedingten Fehlern wie Ausschwitzen oder Substratverschmutzung neigt. Dies im Gegensatz zu ausgehärteten Zusammensetzungen auf Basis von Silangruppen-haltigen organischen Polymere enthaltend Amidin- oder Guanidin-Katalysatoren, wie sie aus dem Stand der Technik bekannt sind, wo katalysatorbedingte Migrationseffekte bekannt sind, wie bereits erwähnt.

Aus der Verwendung als Klebstoff, Dichtstoff oder Beschichtung entsteht ein Artikel, welcher mit einer erfindungsgemässen Zusammensetzung verklebt, abgedichtet oder beschichtet wurde. Beim Artikel handelt es sich insbesondere um ein Bauwerk, insbesondere ein Bauwerk des Hoch- oder Tiefbaus, um ein industriell gefertigtes Gut oder um ein Konsumgut, insbesondere ein Fenster, eine Haushaltmaschine oder ein Transportmittel wie insbesondere ein Automobil, ein Bus, ein Lastkraftwagen, ein Schienenfahrzeug, ein Schiff, ein Flugzeug oder ein Helikopter; oder der Artikel kann ein Anbauteil davon sein.

Weiterhin betrifft die vorliegende Erfindung die Verwendung eines Katalysators der Formel (I), wie er vorhergehend beschrieben worden ist, als Vernetzungskatalysator für härtbare Zusammensetzungen, insbesondere für Silangruppen-haltige härtbare Zusammensetzungen, insbesondere für Zusammensetzungen, wie sie vorhergehend beschrieben worden sind.
In einer bevorzugten Verwendung des Katalysators der Formel (I) ist die feuchtigkeitshärtende Zusmmensetzung im Wesentlichen frei von Organozinn-Verbindungen. Insbesondere beträgt der Zinngehalt der Zusammensetzung weniger als 0.06 Gew.-%, insbesondere weniger als 0.01 Gew.-%.

Ein weiterer Gegenstand der vorliegenden Erfindung ist ein Katalysator der Formel (I a), 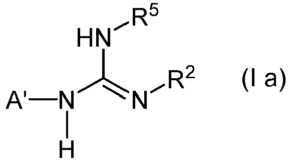 wobei
A' für einen aliphatischen oder cycloaliphatischen oder arylaliphatischen Kohlenwasserstoff-Rest mit 6 bis 30 C-Atomen, welcher gegebenenfalls Ethersauerstoffe enthält, und welcher keine Silangruppen, keine Hydroxylgruppen und keine Aminogruppen aufweist, steht,
und R² und R⁵ die bereits beschriebenen Bedeutungen aufweisen.

Ein Katalysator der Formel (I a) ist nicht oder wenig feuchtigkeitsempfindlich, geruchsarm und in einem einfachen Verfahren in hoher Reinheit herstellbar. Er ist besonders geeignet als Katalysator für die Bildung von Siloxangruppen. Insbesondere geeignet ist er als Vernetzungskatalysator für Silangruppen-haltige Polymere wie insbesondere Polyorganosiloxane mit endständigen Silangruppen oder Silangruppen-haltige organische Polymere wie vorgängig beschrieben. Überraschenderweise migriert er trotz der Abwesenheit von Silangruppen nicht an die Oberfläche eines solchen ausgehärteten Polymers, obwohl er bei der Aushärtung nicht ins Polymer eingebaut werden kann.

Ein weiterer Gegenstand der vorliegenden Erfindung ist ein Katalysator der Formel (I b), 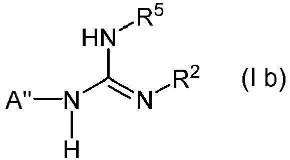 wobei
A" für einen aliphatischen oder cycloaliphatischen oder arylaliphatischen Kohlenwasserstoff-Rest mit 3 bis 30 C-Atomen, welcher frei ist von Silangruppen, gegebenenfalls Ethersauerstoffe enthält und mindestens eine Hydroxylgruppe oder primäre oder sekundäre Aminogruppe aufweist, steht,
und R² und R⁵ die bereits beschriebenen Bedeutungen aufweisen.
Ein Katalysator der Formel (I b) ist nicht oder wenig feuchtigkeitsempfindlich und geruchsarm. Er ist besonders schwerflüchtig und überraschenderweise besonders gut verträglich mit Silangruppen-haltigen Polymeren. Aufgrund der Hydroxylgruppe bzw. der primären oder sekundären Aminogruppe kann er über eine besonders hohe katalytische Aktivität verfügen oder eine besonders gute Wechselwirkung mit einem Silangruppen-haltigen Polymer aufweisen. Überraschenderweise migriert er trotz der Abwesenheit von Silangruppen nicht an die Oberfläche eines solchen ausgehärteten Polymers, obwohl er bei der Aushärtung nicht in ein Silangruppen-haltiges Polymer eingebaut werden kann. Ausserdem eignet sich ein solcher Katalysator zum Aufbau von weiteren Guanidin-funktionellen Verbindungen.
Ein Katalysator der Formel (I b) ist besonders geeignet als Katalysator für die Bildung von Siloxangruppen.
Insbesondere geeignet ist er als Vernetzungskatalysator für Silangruppen-haltige Polymere wie insbesondere Polyorganosiloxane mit endständigen Silangruppen oder Silangruppen-haltige organische Polymere.

Ein weiterer Gegenstand der vorliegenden Erfindung ist ein Katalysator der Formel (I c), 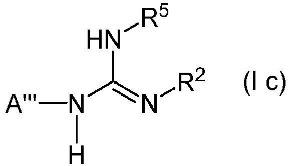 wobei
A'" für einen aliphatischen oder cycloaliphatischen oder arylaliphatischen Kohlenwasserstoff-Rest mit 3 bis 30 C-Atomen, welcher frei ist von Silangruppen, gegebenenfalls Ethersauerstoff enthält und mindestens eine tertiäre Aminogruppe aufweist, steht,
und R² und R⁵ die bereits beschriebenen Bedeutungen aufweisen.
Ein Katalysator der Formel (I c) ist nicht oder wenig feuchtigkeitsempfindlich und geruchsarm. Er verfügt überraschenderweise über eine ganz besonders hohe katalytische Aktivität zur Aushärtungsbeschlenigung von Silangruppen-haltigen Polymeren und ist in diesen sehr gut verträglich. Überraschenderweise migriert er trotz der Abwesenheit von Silangruppen nicht an die Oberfläche eines solchen ausgehärteten Polymers, obwohl er bei der Aushärtung nicht ins Polymer eingebaut werden kann.
Ein Katalysator der Formel (I c) ist besonders geeignet als Katalysator für die Bildung von Siloxangruppen.
Insbesondere geeignet ist er als Vernetzungskatalysator für Silangruppen-haltige Polymere wie insbesondere Polyorganosiloxane mit endständigen Silangruppen oder Silangruppen-haltige organische Polymere.

Geeignete Silangruppen-haltige organische Polymere wurden bereits vorgängig beschrieben.

Ein geeignetes Polyorganosiloxan mit endständigen Silangruppen weist insbesondere die Formel (VI) auf, 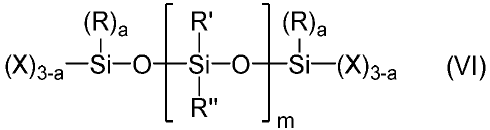 wobei
R, R' und R" unabhängig voneinander jeweils für einen einwertigen Kohlenwasserstoff-Rest mit 1 bis 12 C-Atomen stehen;
X für einen Hydroxyl-Rest oder für einen Alkoxy-, Acetoxy-, Ketoximato-, Amido- oder Enoxy-Rest mit 1 bis 13 C-Atomen steht;
a für 0, 1 oder 2 steht; und
m für eine ganze Zahl im Bereich von 50 bis etwa 2'500 steht.

R steht bevorzugt für Methyl, Vinyl oder Phenyl.
R' und R" stehen bevorzugt unabhängig voneinander jeweils für einen Alkylrest mit 1 bis 5, bevorzugt 1 bis 3 C-Atomen, insbesondere für Methyl.

X steht bevorzugt für einen Hydroxyl-Rest oder für einen Alkoxy- oder Ketoximato-Rest mit 1 bis 6 C-Atomen, insbesondere für einen Hydroxyl-, Methoxy-, Ethoxy-, Methylethylketoximato- oder Methylisobutylketoximato-Rest. Besonders bevorzugt steht X für einen Hydroxylrest.
a steht bevorzugt für 0 oder 1, insbesondere für 0.
Weiterhin ist m bevorzugt so gewählt, dass das Polyorganosiloxan der Formel (VI) bei Raumtemperatur eine Viskosität im Bereich von 100 bis 500'000 mPa·s, insbesondere von 1000 bis 100'000 mPa·s, aufweist.

Solche Polyorganosiloxane sind gut handhabbar und vernetzen mit Feuchtigkeit und/oder Silanvernetzern zu festen Silicon-Polymeren mit elastischen Eigenschaften.
Geeignete kommerziell erhältliche Polyorganosiloxane sind beispielsweise erhältlich von Wacker, Momentive Performance Material, GE Advanced Materials, Dow Corning, Bayer oder Shin Etsu.

Das Polyorganosiloxan mit endständigen Silangruppen kann einen Silanvernetzer enthalten, insbesondere ein Silan der Formel (VII),

(R''')_{q}-Si-(X')_{4-q} (VII)

wobei
R''' für einen einwertigen Kohlenwasserstoff-Rest mit 1 bis 12 C-Atomen steht, X' für einen Hydroxyl-Rest oder für einen Alkoxy-, Acetoxy-, Ketoximato-, Amido- oder Enoxy-Rest mit 1 bis 13 C-Atomen steht; und
q für einen Wert von 0, 1 oder 2, insbesondere für 0 oder 1, steht.

Besonders geeignete Silane der Formel (VII) sind Methyltrimethoxysilan, Ethyltrimethoxysilan, Propytrimethoxysilan, Vinyltrimethoxysilan, Methyltriethoxysilan, Vinyltriethoxysilan, Phenyltriethoxysilan, Tetramethoxysilan, Tetraethoxysilan, Methyltris(methylethylketoximo)silan, Vinyltris(methylethylketoximo)silan und Methyltris(isobutylketoximo)silan.

### Beispiele

Im Folgenden sind Ausführungsbeispiele aufgeführt, welche die beschriebene Erfindung näher erläutern sollen. Selbstverständlich ist die Erfindung nicht auf diese beschriebenen Ausführungsbeispiele beschränkt.

Als "Normklima" wird eine Temperatur von 23±1 °C und eine relative Luftfeuchtigkeit von 50±5% bezeichnet.

**¹H-NMR-Spektren** wurden auf einem Spektrometer des Typs Bruker Ascend 400 bei 400.14 MHz gemessen; die chemischen Verschiebungen δ sind angegeben in ppm relativ zu Tetramethylsilan (TMS). Echte und Pseudo-Kopplungsmuster wurden nicht unterschieden.
**Infrarotspektren** (FT-IR) wurden auf einem mit horizontaler ATR-Messeinheit mit Diamant-Kristall ausgestatteten FT-IR Gerät Nicolet iS5 von Thermo Scientific gemessen. Flüssige Proben wurden unverdünnt als Filme aufgetragen, feste Proben wurden in CH₂Cl₂ gelöst. Die Absorptionsbanden sind in Wellenzahlen (cm⁻¹) angegeben (Messfenster: 4000-650 cm⁻¹).
**Viskositäten** wurden auf einem thermostatisierten Kegel-Platten-Viskosimeter Rheotec RC30 (Kegeldurchmesser 50 mm, Kegelwinkel 1°, Kegelspitze-Platten-Abstand 0.05 mm, Scherrate 10 s⁻¹) gemessen.
Die **Hautbildungszeit** (HBZ) wurde dadurch bestimmt, dass einige Gramm der Zusammensetzung in einer Schichtdicke von ca. 2 mm auf Pappkarton aufgetragen wurden und im Normklima die Zeitdauer gemessen wurde, bis beim leichten Antippen der Oberfläche der Zusammensetzung mittels einer Pipette aus LDPE erstmals keine Rückstände auf der Pipette mehr zurückblieben.
Die Beschaffenheit der **Oberfläche** wurde haptisch geprüft.
Die mechanischen Eigenschaften **Zugfestigkeit, Bruchdehnung** und **E-Modul** (bei 0-5% und bei 0-50% Dehnung) wurden gemäss DIN EN 53504 bei einer Zuggeschwindigkeit von 200 mm/min. gemessen.

### Herstellung von Katalysatoren der Formel (I):

### Vergleichskatalysator K-1: 1-(3-Triethoxysilylpropyl)-2,3-dicyclohexylguanidin

In einem Rundkolben wurden 6.69 g 3-Aminopropyl-triethoxysilan und 5.17 g N,N'-Dicyclohexylcarbodiimid vermischt und die Mischung unter Rühren auf 100 °C erwärmt. In regelmässigen Abständen wurde das Reaktionsgemisch mittels FT-IR-Spektroskopie untersucht. Nach 40 Stunden war die Carbodiimid-Bande bei ca. 2120 cm⁻¹ vollständig verschwunden. Darauf wurde das Reaktionsgemisch im Vakuum von den flüchtigen Bestandteilen befreit. Man erhielt 11.38 g eines farblosen, geruchsarmen Öls.
¹H-NMR (CDCl₃): δ 0.5-0.75 (*m*, 2 H, CH₂Si), 1.1-1.4 (*m*, 21 H), 1.55-2.0 (*m*, 10 H), 2.95-3.1 (*m,* 2 H, NCH^{Cy}), 3.1-3.3 *(m,* 2 H, CH₂N), 3.85 (*q*, 6 H, CH₂O). FT-IR: 2972, 2924, 2851, 1641 (C=N), 1496, 1447, 1389, 1363, 1326, 1297, 1237, 1165, 1101, 1074, 953, 888, 773.

### Vergleichskatalysator K-2: 1-(3-Trimethoxysilylpropyl)-2,3-dicyclohexylguanidin

In einem Rundkolben wurden 6.42 g 3-Aminopropyl-trimethoxysilan und 5.73 g N,N'-Dicyclohexylcarbodiimid vermischt und die Mischung unter Rühren auf 110 °C erwärmt. In regelmässigen Abständen wurde das Reaktionsgemisch mittels FT-IR-Spektroskopie untersucht. Nach 24 Stunden war die Carbodiimid-Bande bei ca. 2120 cm⁻¹ vollständig verschwunden. Darauf wurde das Reaktionsgemisch im Vakuum von den flüchtigen Bestandteilen befreit. Man erhielt 10.66 g eines hellgelben, geruchsarmen Öls.
¹H-NMR (CDCl₃): δ 0.5-0.75 (*m*, 2 H, CH₂Si), 1.1-1.4 (*m*, 12 H), 1.55-2.0 (*m*, 10 H), 2.95-3.1 (*m*, 2 H, NCH^{Cy}), 3.1-3.3 (*m*, 2 H, CH₂N), 3.55 (*s*, 9 H, CH₃O). FT-IR: 3405 (N-H), 2923, 2849, 1639 (C=N), 1498, 1448, 1327, 1305, 1237, 1189, 1081, 979, 889, 814, 787, 713.

### Vergleichskatalysator K-3: 1-(N-(3-Trimethoxysilylpropyl)-2-aminoethyl)-2,3-dicyclohexylguanidin

In einem Rundkolben wurden 6.11 g N-(2-Aminoethyl)-3-aminopropyl-trimethoxysilan (Silquest® A-1120, von Momentive) und 5.16 g N,N'-Dicyclohexylcarbodiimid vermischt und die Mischung unter Rühren auf 120 °C erwärmt. In regelmässigen Abständen wurde das Reaktionsgemisch mittels FT-IR-Spektroskopie untersucht. Nach 25 Stunden war die Carbodiimid-Bande bei ca. 2120 cm⁻¹ vollständig verschwunden. Man erhielt ein farbloses, geruchsarmes Öl.
¹H-NMR (CDCl₃): δ 0.6-0.7 (*m,* 2 H, CH₂Si), 1.0-1.5 (*m,* 10 H), 1.55-2.0 (*m,* 12 H), 2.60 (*m*, 2 H, CH₂N), 2.75 (*m*, 2 H, CH₂N), 2.9 (*m*, 1 H, NCH^{Cy}), 3.11 (*m*, 2 H, CH₂N), 3.23 *(m,* 1 H, NCH^{Cy}), 3.5-3.6 (*br s,* 9 H, CH₃O).
FT-IR: 3233, 2924, 2839, 1637 (C=N), 1605, 1538, 1448, 1409, 1364, 1330, 1269, 1257, 1189, 1079, 889, 813, 781.

### Vergleichskatalysator K-4: 1-(N-(3-Triethoxysilylpropyl)-2-aminoethyl)-2,3-dicyclohexylguanidin

In einem Rundkolben wurden 7.27 g N-(2-Aminoethyl)-3-aminopropyl-triethoxysilan (Geniosil® GF-94, von Wacker Chemie) und 5.16 g N,N'-Dicyclohexylcarbodiimid vermischt und die Mischung unter Rühren auf 120 °C erwärmt. In regelmässigen Abständen wurde das Reaktionsgemisch mittels FT-IR-Spektroskopie untersucht. Nach 12 Stunden war die Carbodiimid-Bande bei ca. 2120 cm⁻¹ vollständig verschwunden. Man erhielt ein farbloses, geruchsarmes Öl.
¹H-NMR (CDCl₃): δ 0.6-0.7 (*m*, 2 H, CH₂Si), 1.05-1.45 (*m*, 19 H), 1.55-2.1 (*m*, 12 H), 2.60 (*m*, 2 H, CH₂N), 2.75 (*m*, 2 H, CH₂N), 2.9 (*m*, 1 H, NCH^{Cy}), 3.11 (*m*, 2 H, CH₂N), 3.23 (*m*, 1 H, NCH^{Cy}), 3.70-3.85 (*m*, 6 H, OCH₂C*H*₃).
FT-IR: 3232, 2972, 2924, 2851, 1640 (C=N), 1605, 1544, 1448, 1389, 1344, 1269, 1257, 1165, 1101, 1074, 954, 889, 772, 671.

### Katalysator K-5: 1-Hexyl-2,3-dicyclohexylguanidin

In einem Rundkolben wurden 2.05 g n-Hexylamin und 3.68 g N,N'-Dicyclohexylcarbodiimid vermischt und die Mischung unter Rühren auf 120 °C erwärmt. In regelmässigen Abständen wurde das Reaktionsgemisch mittels FT-IR-Spektroskopie untersucht. Nach 17 Stunden war die Carbodiimid-Bande bei ca. 2120 cm⁻¹ vollständig verschwunden. Darauf wurde das Reaktionsgemisch im Vakuum von den flüchtigen Bestandteilen befreit. Man erhielt 5.20 g eines farblosen, geruchsarmen Öls.
¹H-NMR (CDCl₃): δ 0.9 (*t*, 3 H, C*H*₃CH₂), 1.1-1.25 (*m,* 6 H), 1.25-1.4 (*m,* 10 H), 1.45-1.65 (*m,* 4 H), 1.7-1.8 (*m,* 4 H), 1.85-1.95 (*m,* 4 H), 2.95-3.05 (*m,* 2 H, CH₂N), 3.1-3.3 (*m*, 2 H, NCH^{Cy}).
FT-IR: 3305 (N-H), 2952, 2850, 1636 (C=N), 1495, 1448, 1361, 1322, 1253, 1147, 1112, 1031, 977, 888, 723.

### Katalysator K-6: 1-Hexyl-2,3-diisopropylguanidin

In einem Rundkolben wurden 2.93 g n-Hexylamin und 2.89 g N,N'-Diisopropylcarbodiimid vermischt und die Mischung unter Rühren auf 120 °C erwärmt. In regelmässigen Abständen wurde das Reaktionsgemisch mittels FT-IR-Spektroskopie untersucht. Nach 4.5 Stunden war die Carbodiimid-Bande bei ca. 2120 cm⁻¹ vollständig verschwunden. Darauf wurde das Reaktionsgemisch im Vakuum von den flüchtigen Bestandteilen befreit. Man erhielt 4.96 g eines hellgelben, geruchsarmen Öls.
FT-IR: 3304 (N-H), 2959, 2926, 2857, 1633 (C=N), 1510, 1466, 1378, 1363, 1336, 1172, 1125, 723.

### Katalysator K-7: 1-Benzyl-2,3-dicyclohexylguanidin

In einem Rundkolben wurden 6.43 g Benzylamin und 10.32 g N,N'-Dicyclohexylcarbodiimid vermischt und die Mischung unter Rühren auf 120 °C erwärmt. In regelmässigen Abständen wurde das Reaktionsgemisch mittels FT-IR-Spektroskopie untersucht. Nach 70 Stunden war die Carbodiimid-Bande bei ca. 2120 cm⁻¹ vollständig verschwunden. Darauf wurde das Reaktionsgemisch im Vakuum von den flüchtigen Bestandteilen befreit. Man erhielt 15.67 g eines hellgelben, geruchsarmen Öls.
¹H-NMR (CDCl₃): δ 1.1-1.5 (*m*, 10 H), 1.55-2.0 (*m*, 10 H), 3.11 (*m*, 2 H, NCH^{Cy}), 3.6 (*br s,* 1 H, NH), 4.31 (*m,* 2 H, C*H*₂Ph), 4.37 (*br s,* 1 H, NH), 7.15-7.35 (*m*, 5 H, Ph-H).
FT-IR: 3432, 3270, 3060, 3025, 2923, 2849, 1633 (C=N), 1493, 1448, 1335, 1328, 1253, 1236, 1188, 1146, 1112, 1072, 1028, 1002, 977, 888, 860, 802, 730, 696.

### Katalysator K-8: 1-(2-Ethylhexyl)-2,3-dicyclohexylguanidin

In einem Rundkolben wurden 7.76 g 2-Ethylhexylamin und 10.32 g N,N'-Dicyclohexylcarbodiimid vermischt und die Mischung unter Rühren auf 120 °C erwärmt. In regelmässigen Abständen wurde das Reaktionsgemisch mittels FT-IR-Spektroskopie untersucht. Nach 70 Stunden war die Carbodiimid-Bande bei ca. 2120 cm⁻¹ vollständig verschwunden. Darauf wurde das Reaktionsgemisch im Vakuum von den flüchtigen Bestandteilen befreit. Man erhielt 16.78 g eines orangegelben, geruchsarmen Öls.
¹H-NMR (CDCl₃): δ 0.88 (*m,* 6 H, CH₃), 1.06-1.2 (*m,* 7 H), 1.2-1.4 (*m,* 10 H), 1.4-1.5 (*m*, 2 H), 1.55-2.0 (*m*, 10 H), 2.86-3.0 (*m*, 2 H, CH₂N), 3.0-3.2 (*m*, 2 H, NCH^{Cy}).
FT-IR: 3440, 2954, 2923, 2851, 1644 (C=N), 1493, 1448, 1361, 1335, 1255, 1236, 1188, 1145, 1090, 1050, 1027, 978, 927, 888, 845, 769, 726.

### Katalysator K-9: 1-(5-Hydroxy-3-oxa-pentyl)-2,3-dicyclohexylguanidin

In einem Rundkolben wurden 3.55 g 2-(2-Aminoethoxy)ethanol (Diglycolamine® Agent, von Huntsman) und 6.81 g N,N'-Dicyclohexylcarbodiimid vermischt und die Mischung unter Rühren auf 120 °C erwärmt. In regelmässigen Abständen wurde das Reaktionsgemisch mittels FT-IR-Spektroskopie untersucht. Nach 24 Stunden war die Carbodiimid-Bande bei ca. 2120 cm⁻¹ vollständig verschwunden. Darauf wurde das Reaktionsgemisch im Vakuum von den flüchtigen Bestandteilen befreit. Man erhielt 10.29 g eines hellgelben, geruchsarmen Öls.
¹H-NMR (CDCl₃): δ 1.05-1.3 und 1.3-1.45 (2 x *m*, 10 H, CH₂), 1.54-1.78 (*m*, 8 H), 1.88-2.0 (*m,* 4 H), 3.13 (*t,* 2 H, CH₂N), 3.69 (*m,* 4 H, CH₂O), 3.81 (*t,* 2 H, OC*H*₂CH₂N).
FT-IR: 3355 (O-H), 2922, 2849, 1617 (C=N), 1520, 1447, 1340, 1257, 1240, 1117, 1066, 888, 717.

### Katalysator K-10: 1-(3-Aminopropyl)-2,3-dicyclohexylguanidin

In einem Rundkolben wurden 2.50 g 1,3-Diaminopropan und 6.89 g N,N'-Dicyclohexylcarbodiimid vermischt und die Mischung unter Rühren auf 120 °C erwärmt. In regelmässigen Abständen wurde das Reaktionsgemisch mittels FT-IR-Spektroskopie untersucht. Nach 1 Stunde war die Carbodiimid-Bande bei ca. 2120 cm⁻¹ vollständig verschwunden. Darauf wurde das Reaktionsgemisch im Vakuum von den flüchtigen Bestandteilen befreit. Man erhielt 9.36 g eines hellgelben, geruchsarmen Öls.
¹H-NMR (CDCl₃): δ 1.05-1.2 und 1.25-1.40 (2 x *m*, 10 H), 1.54-1.78 (*m*, 10 H), 1.88-2.0 (*m,* 4 H), 2.73 (*m,* 2 H), 3.12(*m,* 2 H), 3.22 (br s, 2 H).
FT-IR: 3371 (N-H), 2921, 2849, 1627 (C=N), 1502, 1447, 1324, 1238, 1147, 1111, 888, 713.

### Katalysator K-11: 1-(3-Cyclohexylaminopropyl)-2,3-dicyclohexylguanidin

In einem Rundkolben wurden 25.78 g 3-(Cyclohexylamino)propylamin (von BASF) und 30.95 g N,N'-Dicyclohexylcarbodiimid vermischt und die Mischung unter Rühren auf 120 °C erwärmt. In regelmässigen Abständen wurde das Reaktionsgemisch mittels FT-IR-Spektroskopie untersucht. Nach 48 Stunden war die Carbodiimid-Bande bei ca. 2120 cm⁻¹ vollständig verschwunden. Darauf wurde das Reaktionsgemisch im Vakuum von den flüchtigen Bestandteilen befreit. Man erhielt 54.4 g eines hellgelben, geruchsarmen Öls.
FT-IR: 3283 (N-H), 2921, 2849, 1634 (C=N), 1493, 1447, 1343, 1322, 1256, 1145, 1111, 888, 713.

### Katalysator K-12: 1-(3-Cyclohexylaminopropyl)-2,3-diisopropylguanidin

In einem Rundkolben wurden 34.38 g 3-(Cyclohexylamino)propylamin (von BASF) und 25.24 g N,N'-Diisopropylcarbodiimid vermischt und die Mischung unter Rühren auf 120 °C erwärmt. In regelmässigen Abständen wurde das Reaktionsgemisch mittels FT-IR-Spektroskopie untersucht. Nach 2 Stunden war die Carbodiimid-Bande bei ca. 2120 cm⁻¹ vollständig verschwunden. Darauf wurde das Reaktionsgemisch im Vakuum von den flüchtigen Bestandteilen befreit. Man erhielt 56.50 g eines hellgelben, geruchsarmen Öls.
FT-IR: 3292 (N-H), 2960, 2924, 2852, 1633 (C=N), 1505, 1448, 1361, 1329, 1176, 1125, 889, 714.

### Katalysator K-13: 1-(6-Amino-2,2(4),4-trimethyl-hexyl)-2,3-dicyclohexylguanidin und 1-(6-Amino-3,3(5),5-trimethyl-hexyl)-2,3-dicyclohexylguanidin

In einem Rundkolben wurden 16.62 g Vestamin® TMD (Gemisch von 2,2,4- und 2,4,4-Trimethyl-1,6-hexamethylendiamin, von Evonik) und 20.63 g N,N'-Dicyclohexylcarbodiimid vermischt und die Mischung unter Rühren auf 120 °C erwärmt. In regelmässigen Abständen wurde das Reaktionsgemisch mittels FT-IR-Spektroskopie untersucht. Nach 3 Stunden war die Carbodiimid-Bande bei ca. 2120 cm⁻¹ vollständig verschwunden. Darauf wurde das Reaktionsgemisch im Vakuum von den flüchtigen Bestandteilen befreit. Man erhielt 36.5 g eines hellgelben, geruchsarmen Öls.
FT-IR: 3281 (N-H), 2923, 2850, 1635 (C=N), 1496, 1463, 1448, 1362, 1325, 1284, 1237, 1188, 1146, 1112, 1090, 1071, 1051, 1027, 977, 888, 860, 845, 804, 785, 714.

### Katalysator K-14: 1-(5-Amino-2-methylpentyl)-2,3-dicyclohexylguanidin und 1-(5-Amino-4-methylpentyl)-2,3-dicyclohexylguanidin

In einem Rundkolben wurden 12.20 g 2-Methylpentan-1,5-diamin (MPMD; von Invista) und 20.63 g N,N'-Dicyclohexylcarbodiimid vermischt und die Mischung unter Rühren auf 120 °C erwärmt. In regelmässigen Abständen wurde das Reaktionsgemisch mittels FT-IR-Spektroskopie untersucht. Nach 3 Stunden war die Carbodiimid-Bande bei ca. 2120 cm⁻¹ vollständig verschwunden. Darauf wurde das Reaktionsgemisch im Vakuum von den flüchtigen Bestandteilen befreit. Man erhielt 32.3 g eines hellgelben, geruchsarmen Öls.
FT-IR: 3304 (N-H), 2922, 2849, 1636 (C=N), 1495, 1462, 1447, 1361, 1324, 1285, 1237, 1188, 1145, 1112, 1071, 1051, 1027, 977, 926, 888, 859, 845, 802, 786, 715.

### Katalysator K-15: 1-(3-(N,N-Dimethylamino)propyl)-2,3-dicyclohexylguanidin

In einem Rundkolben wurden 6.64 g 3-(N,N'-Dimethylamino)propylamin und 10.32 g N,N'-Dicyclohexylcarbodiimid vermischt und die Mischung unter Rühren auf 120 °C erwärmt. In regelmässigen Abständen wurde das Reaktionsgemisch mittels FT-IR-Spektroskopie untersucht. Nach 48 Stunden war die Carbodiimid-Bande bei ca. 2120 cm⁻¹ vollständig verschwunden. Darauf wurde das Reaktionsgemisch im Vakuum von den flüchtigen Bestandteilen befreit. Man erhielt 15.43 g eines orangegelben, geruchsarmen Öls.
¹H-NMR (CDCl₃): δ 1.05-1.38 (*m*, 10 H), 1.54-1.65 (*m*, 2 H), 1.65-1.80 (*m*, 6 H), 1.80-2.0 (*m,* 4 H), 2.0 (s, 6 H, NMe₂), 2.30 (*m,* 2 H, C*H*₂NMe₂), 3.06-3.25 (*m*, 4 H).
FT-IR: 3291, 2922, 2850, 2814, 1635 (C=N), 1494, 1447, 1361, 1322, 1256, 1236, 1147, 1098, 1068, 1040, 996, 977, 888, 845, 785, 713.

### Katalysator K-16: 1-(ω-Methoxy-polyoxypropylen-polyoxyethylen)-2,3-dicyclohexylguanidin mit einem Molekulargewicht von etwa 810 g/mol

In einem Rundkolben wurden 14.32 g Polyoxypropylenmonoamin mit einem mittleren Molekukargewicht von ca. 600 g/mol (Jeffamine® M-600 von Huntsman, Amingehalt ca. 1.7 meq/g) und 4.14 g N,N'-Dicyclohexylcarbodiimid vermischt und die Mischung unter Rühren auf 120 °C erwärmt. In regelmässigen Abständen wurde das Reaktionsgemisch mittels FT-IR-Spektroskopie untersucht. Nach 72 Stunden war die Carbodiimid-Bande bei ca. 2120 cm⁻¹ vollständig verschwunden. Man erhielt 18.45 g eines hellgelben, geruchlosen Öls. FT-IR: 3367 (N-H), 2969, 2926, 2854, 1640 (C=N), 1449, 1372, 1342, 1297, 1255, 1100, 1014, 925, 889, 861, 715.

### Vergleichskatalysator K-17: 1,2,3-Tricyclohexylguanidin

In einem Rundkolben wurden 7.99 g Cyclohexylamin und 15.83 g N,N'-Dicyclohexylcarbodiimid vermischt und die Mischung unter Rühren auf 120 °C erwärmt. In regelmässigen Abständen wurde das Reaktionsgemisch mittels FT-IR-Spektroskopie untersucht. Nach 48 Stunden war die Carbodiimid-Bande bei ca. 2120 cm⁻¹ vollständig verschwunden. Darauf wurde das Reaktionsgemisch im Vakuum von den flüchtigen Bestandteilen befreit. Man erhielt 23.44 g eines geruchsarmen braunen Feststoffs.
¹H-NMR (CDCl₃): δ 1.05-1.38 (*m*, 16 H), 1.54-1.65 (*m*, 4 H), 1.65-1.80 (*m*, 6 H), 1.8-2.0 (*m*, 6 H), 3.06-3.25 (br *s*, 3 H).
FT-IR: 2923, 2851, 1643 (C=N), 1491, 1448, 1363, 1335, 1322, 1281, 1254, 1234, 1145, 1111, 978, 927, 846, 749, 720, 668.

### Katalysator K-18: 1-(3-(3-(N,N-Dimethylamino)propyl)aminopropyl)-2,3-dicyclohexylguanidin

In einem Rundkolben wurden 9.40 g N¹-((3-Dimethylamino)propyl)-1,3-diaminopropan und 11.65 g N,N'-Dicyclohexylcarbodiimid vermischt und die Mischung unter Rühren auf 120 °C erwärmt. In regelmässigen Abständen wurde das Reaktionsgemisch mittels FT-IR-Spektroskopie untersucht. Nach 3 Stunden war die Carbodiimid-Bande bei ca. 2120 cm⁻¹ vollständig verschwunden. Man erhielt ein gelbes, geruchsarmes Öl.
¹H-NMR (CDCl₃): δ 1.05-1.38 (*m,* 10 H), 1.54-1.70 (*m,* 10 H), 1.80-2.0 (*m*, 4 H), 2.21 (s, 6 H, NMe₂), 2.30 (*m*, 2 H, C*H*₂NMe₂), 2.58-2.70 (*m*, 4 H, CH₂NH), 3.06-3.25 (*m,* 4 H).
FT-IR: 3270, 2922, 2850, 2813, 2784, 2763, 1633 (C=N), 1495, 1447, 1359, 1335, 1321, 1257, 1237, 1097, 1041, 977, 888, 842, 785, 719.

### Katalysator Ref-1: 1-Hexyl-2,3-bis(2,6-diisopropylphenyl)guanidin

In einem Rundkolben wurden 1.20 g n-Hexylamin und 4.01 g Bis(2,6-diisopropylphenyl)carbodiimid (Stabilisator 7000 von Raschig) vermischt und die Mischung unter Rühren auf 120 °C erwärmt. In regelmässigen Abständen wurde das Reaktionsgemisch mittels FT-IR-Spektroskopie untersucht. Nach 2 Stunden war die Carbodiimid-Bande bei ca. 2120 cm⁻¹ vollständig verschwunden. Man erhielt ein gelbes, geruchsarmes Öl.
FT-IR: 3442, 3397, 2957, 2925, 2866, 1637 (C=N), 1585, 1513, 1459, 1434, 1391, 1361, 1324, 1300, 1255, 1198, 1179, 1126, 1098, 1058, 1044, 956, 934, 895, 831, 804, 764, 699.

### Katalysator Ref-2: 1-Phenyl-2,3-diisopropylguanidin

In einem Reagensglas wurden 2.24 g Anilin und 2.52 g N,N'-Diisopropylcarbodiimid (DIC) vermischt und die Mischung im Mikrowellengerät während 10 Minuten bei einer Temperatur von 220 °C und einem Druck von 150 Pa zur Reaktion gebracht. Das erhaltene Produkt wurde aus einer Mischung von Ethylacetat und Heptan im Verhältnis 1:1 umkristallisiert. Man erhielt 2.77 g eines weissen Feststoffs.
¹H-NMR (CDCl₃): δ 1.08 (*d*, 24 H, C*H*₃), 3.65 (m, 2 H, *CH* aliph.), 6.75-6.80 (m, 2 H, *CH* arom.), 6.82-6.88 (m, 1 H, *CH* arom.), 7.14-7.20 (m, 2 H, *CH* arom.). FT-IR: 3346, 3055, 2966, 2930, 2869, 1705, 1630 (C=N), 1587, 1532, 1500, 1487, 1464, 1455, 1444, 1383, 1364, 1313, 1225, 1174, 1124, 1069, 1030, 996, 941, 898, 864, 831, 810, 755, 698.

### Herstellung von Silangruppen-haltigen Polvethern:

### Polymer STP-1:

Unter Feuchtigkeitsausschluss wurden 1000 g Polyol Acclaim® 12200 (Polyoxypropylendiol mit niedrigem Ungesättigtheitsgrad, von Bayer; OH-Zahl 11.0 mg KOH/g), 43.6 g Isophorondiisocyanat (IPDI; Vestanat® IPDI, von Evonik), 126.4 g Diisodecylphthalat (DIDP) und 0.1 g Bismuttris(neodecanoat) (10 Gew.-% in DIDP) unter stetigem Rühren auf 90 °C aufgeheizt und auf dieser Temperatur belassen, bis der titrimetrisch bestimmte Gehalt an freien Isocyanatgruppen einen stabilen Wert von 0.63 Gew.-% erreicht hatte. Anschliessend wurden 63.0 g N-(3-Trimethoxysilylpropyl)-amino-bernsteinsäurediethylester (Addukt aus 3-Aminopropyltrimethoxysilan und Maleinsäurediethylester; hergestellt nach den Angaben in US 5,364,955) eingemischt und die Mischung bei 90 °C solange gerührt, bis mittels FT-IR-Spektroskopie kein freies Isocyanat mehr nachgewiesen wurde. Der so erhaltene Trimethoxysilangruppen-haltige Polyether mit einem Silan-Equivalentgewicht von ca. 6880 g/Eq (aus den Einsatzmengen berechnet) wurde auf Raumtemperatur abgekühlt und unter Ausschluss von Feuchtigkeit aufbewahrt.

### Polymer STP-2:

Unter Feuchtigkeitsausschluss wurden 1000 g Polyol Acclaim® 12200 (Polyoxypropylendiol mit niedrigem Ungesättigtheitsgrad, von Bayer; OH-Zahl 11.0 mg KOH/g), 43.6 g Isophorondiisocyanat (IPDI; Vestanat® IPDI, von Evonik), 126.4 g Diisodecylphthalat (DIDP) und 0.1 g Bismuttris(neodecanoat) (10 Gew.-% in DIDP) unter stetigem Rühren auf 90 °C aufgeheizt und auf dieser Temperatur belassen, bis der titrimetrisch bestimmte Gehalt an freien Isocyanatgruppen einen stabilen Wert von 0.64 Gew.-% erreicht hatte. Anschliessend wurden 70.6 g N-(3-Triethoxysilylpropyl)-amino-bernsteinsäurediethylester (Addukt aus 3-Aminopropyltriethoxysilan und Maleinsäurediethylester) eingemischt und die Mischung bei 90 °C solange gerührt, bis mittels FT-IR-Spektroskopie kein freies Isocyanat mehr nachgewiesen wurde. Der so erhaltene Triethoxysilangruppen-haltige Polyether mit einem Silan-Equivalentgewicht von ca. 6920 g/Eq (aus den Einsatzmengen berechnet) wurde auf Raumtemperatur abgekühlt und unter Ausschluss von Feuchtigkeit aufbewahrt.

### Verwendete kommerzielle Katalysatoren und Abkürzungen dafür:

- DBU: 1,8-Diazabicyclo[5.4.0]undec-7-en
- TMG: 1,1,3,3-Tetramethylguanidin
- DHA: N,N'-Di-n-hexylacetamidin
- IBAY: Tyzor® IBAY, Bis(ethylacetoacetato)diisobutoxy-titan(IV), von Dorf Ketal
- MTHP: 2-Methyl-1,4,5,6-tetrahydropyrimidin
- TMTHP: 2,5,5-Trimethyl-1,4,5,6-tetrahydropyrimidin
- DBTDL: Dibutylzinn(IV)dilaurat

### Zusammensetzungen auf Basis von Silangruppen-haltigen Polymeren:

Vergleichsbeispiele sind in den Tabellen 1 bis 10 mit (Ref) gekennzeichnet.

### Zusammensetzungen B1 bis B22 und Vergleiche B23 bis B31:

Eine Zusammensetzung aus 96.5 g Polymer STP-1, 0.5 g Vinyltrimethoxysilan und 3.0 g 3-Aminopropyltrimethoxysilan wurde mit verschiedenen Katalysatoren in der angegebenen Menge gemäss Tabelle 1 vermengt und die Mischung auf Viskosität und Hautbildungszeit (HBZ) im Normklima, vor und nach Lagerung, geprüft. Die Hautbildungszeit dient dabei als Mass für die Aktivität des Katalysators in Bezug auf die Vernetzungsreaktion der Silangruppen, d.h. für die Vernetzungsgeschwindigkeit; die Veränderung der Viskosität und der Hautbildungszeit nach Lagerung sind ein Mass für die Lagerstabilität. Weiterhin wurde die applizierte Mischung nach 24 Stunden im Normklima daraufhin geprüft, ob die Oberfläche wie gewünscht trocken war oder sich ein schmieriger Film gebildet hatte, was ein Anzeichen für das Ausschwitzen des Katalysators aufgrund schlechter Verträglichkeit mit dem ausgehärteten Kunststoff ist, und/oder ob die Oberfläche klebrig war, was ein Anzeichen für eine unvollständige Aushärtung ist. Weiterhin wurde von der Mischung ein Film von 2 mm Dicke hergestellt, während 7 Tagen im Normklima aushärten lassen und auf mechanische Eigenschaften geprüft. Die Ergebnisse sind in Tabelle 1 und 2 wiedergegeben. "Zus." steht für "Zusammensetzung".

**Tabelle 1:**

| **Zus.** | **Katalysator** | **Menge** | **Konzentration¹** | **Viskosität [Pa·s] frisch gelagert² Zunahme** | | | **HBZ frisch gelagert²** | |
|---|---|---|---|---|---|---|---|---|
| **B1** | K-1 | 0.41 g | 1.0 | 19.8 | 23.6 | 19% | 21' | 25' |
| **B2** | K-2 | 0.37 g | 1.0 | 20.2 | 22.3 | 10% | 24' | 28' |
| **B3** | K-1 | 0.21 g | 0.5 | 23.6 | 28.0 | 19% | 9' | 27' |
| | IBAY | 0.22 g | 0.5 | | | | | |
| **B4** | K-2 | 0.19 g | 0.5 | 24.4 | 30.5 | 25% | 12' | 18' |
| | IBAY | 0.22 g | 0.5 | | | | | |
| **B5** | K-3 | 0.80 g | 1.9 | 27.2 | 32.4 | 19% | 27' | 33' |
| **B6** | K-4 | 0.87 g | 1.9 | 26.8 | 34.1 | 27% | 30' | 32' |
| **B7** | K-5 | 0.56 g | 1.9 | 37.6 | 37.7 | 0.3% | 12' | 13' |
| **B8** | K-5 | 0.15 g | 0.5 | 48.0 | 50.6 | 5% | 20' | 25' |
| | IBAY | 0.22 g | 0.5 | | | | | |
| **B9** | K-6 | 0.42 g | 1.9 | 24.1 | 27.0 | 12% | 15' | 18' |
| **B10** | K-7 | 0.58 g | 1.9 | 27.8 | 30.0 | 8% | 20' | 19' |
| **B11** | K-8 | 0.62 g | 1.9 | 26.2 | 29.4 | 12% | 16' | 17' |
| **B12** | K-9 | 0.57 g | 1.9 | 26.1 | 31.5 | 21% | 13' | 16' |
| **B13** | K-9 | 0.15 | 0.5 | 35.0 | 39.4 | 11.2% | 21' | 28' |
| | IBAY | g | 0.5 | | | | | |
| **B14** | K-10 | 0.51 g | 1.9 | 21.4 | 28.4 | 33% | 12' | 13' |
| **B15** | K-11 | 0.66 g | 1.9 | 24.1 | 27.3 | 13% | 5' | 13' |
| **B16** | K-12 | 0.52 g | 1.9 | 22.8 | 28.0 | 23% | 9' | 13' |
| **B17** | K-13 | 0.67 g | 1.9 | 28.4 | 39.9 | 41% | 11' | 7' |
| **B18** | K-14 | 0.59 g | 1.9 | 27.7 | 40.1 | 45% | 14' | 8' |
| **B19** | K-15 | 0.56 g | 1.9 | 24.3 | 25.7 | 6% | 6' | 7' |
| **B20** | K-15 | | 0.5 | 50.9 | 52.0 | 2% | 12' | 17' |
| | IBAY | 0.15 g | 0.5 | | | | | |
| **B21** | K-17³ | 0.57 g | 1.9 | 26.0 | 27.2 | 4% | 13' | 14' |
| **B22** | K-18 | 0.67 g | 1.9 | 21.8 | 26.9 | 23% | 8' | 12' |
| **B23** (Ref) | DBU | 0.28 g | 1.9 | 27.2 | 36.9 | 36% | 25' | 29' |
| **B24** (Ref) | TMG | 0.21 g | 1.9 | 22.3 | 24.6 | 10% | 65' | 75' |
| **B25** (Ref) | DHA | 0.42 g | 1.9 | 24.8 | 32.6 | 31% | 40' | 48' |
| **B26** (Ref) | IBAY | 0.87 g | 2.0 | 24.8 | 30.2 | 22% | 45' | 2h |
| **B27** (Ref) | DBU | 0.07 g | 0.5 | 24.9 | 28.1 | 13% | 15' | 55' |
| | IBAY | 0.22 g | 0.5 | | | | | |
| **B28** (Ref) | TMG | 0.06 g | 0.5 | 23.2 | 25.0 | 8% | 37' | 111' |
| | IBAY | 0.22 g | 0.5 | | | | | |
| **B29** (Ref) | - | - | - | 24.8 | 28.9 | 17% | 87' | 110' |
| **B30** (Ref) | Ref-1 | 0.85 g | 1.9 | 22.3 | 22.4 | 0% | 95' | 100' |
| **B31** (Ref) | Ref-2³ | 0.40 g | 1.9 | 20.5 | 21.8 | 6% | 108' | 107' |

| | | | | | | | | |
|---|---|---|---|---|---|---|---|---|
| ¹mmol Amidin- oder Guanidin-Gruppen bzw. Metallatome auf 100 g Silangruppen-haltiger Polyether. ²während 7 Tagen bei 60 °C in geschlossenem Gebinde. ³als Lösung (20 Gew.-%) in N-Ethylpyrrolidon. | | | | | | | | |

**Tabelle 2:**

| **Zus.** | **Oberfläche nach 24h** | **Zugfestigkeit** | **Bruchdehnung** | **E-Modul** | |
|---|---|---|---|---|---|
| | | | | **0-5%** | **0-50%** |
| **B1** | trocken | 0.59 MPa | 77% | 1.18 MPa | 0.77 MPa |
| **B2** | trocken | 0.57 MPa | 72% | 1.17 MPa | 0.78 MPa |
| **B3** | trocken | 0.63 MPa | 90% | 1.13 MPa | 0.75 MPa |
| **B4** | trocken | 0.54 MPa | 69% | 1.17 MPa | 0.76 MPa |
| **B5** | trocken | 0.78 MPa | 116% | 1.11 MPa | 0.80 MPa |
| **B6** | trocken | 0.73 MPa | 98% | 1.08 MPa | 0.81 MPa |
| **B7** | trocken | 0.60 MPa | 76% | 1.18 MPa | 0.81 MPa |
| **B8** | trocken | 0.59 MPa | 73% | 1.26 MPa | 0.81 MPa |
| **B9** | trocken | 0.57 MPa | 75% | 1.14 MPa | 0.76 MPa |
| **B10** | trocken | 0.74 MPa | 114% | 0.98 MPa | 0.76 MPa |
| **B11** | trocken | 0.69 MPa | 105% | 1.08 MPa | 0.76 MPa |
| **B12** | trocken | 0.61 MPa | 78% | 1.05 MPa | 0.75 MPa |
| **B13** | trocken | 0.68 MPa | 95% | 1.17 MPa | 0.80 MPa |
| **B14** | trocken | 0.70 MPa | 98% | 1.04 MPa | 0.78 MPa |
| **B15** | trocken | 0.69 MPa | 97% | 1.07 MPa | 0.78 MPa |
| **B16** | trocken | 0.70 MPa | 95% | 1.04 MPa | 0.79 MPa |
| **B17** | trocken | 0.72 MPa | 102% | 1.23 MPa | 0.79 MPa |
| **B18** | trocken | 0.70 MPa | 91% | 1.20 MPa | 0.81 MPa |
| **B19** | trocken | 0.73 MPa | 105% | 1.11 MPa | 0.78 MPa |
| **B20** | trocken | 0.65 MPa | 87% | 1.24 MPa | 0.81 MPa |
| **B21** | trocken | 0.59 MPa | 73% | 1.19 MPa | 0.81 MPa |
| **B22** | trocken | 0.63 MPa | 84% | 1.23 MPa | 0.81 MPa |
| **B23** (Ref) | schmierig | 0.58 MPa | 72% | 1.16 MPa | 0.77 MPa |
| **B24** (Ref) | klebrig | 0.62 MPa | 90% | 1.19 MPa | 0.75 MPa |
| **B25** (Ref) | leicht klebrig | 0.63 MPa | 82% | 1.10 MPa | 0.78 MPa |
| **B26** (Ref) | klebrig | 0.59 MPa | 91% | 1.05 MPa | 0.69 MPa |
| **B27** (Ref) | leicht schmierig | 0.60 MPa | 81% | 1.11 MPa | 0.76 MPa |
| **B28** (Ref) | leicht klebrig | 0.69 MPa | 100% | 1.13 MPa | 0.76 MPa |
| **B29** (Ref) | trocken | 0.68 MPa | 112% | 1.02 MPa | 0.69 MPa |
| **B30** (Ref) | trocken | 0.59 MPa | 79% | 1.17 MPa | 0.74 MPa |
| **B31** (Ref) | trocken | 0.76 MPa | 117% | 0.79 MPa | 0.75 MPa |

### Zusammensetzungen B32 bis B49 und Vergleiche B50 bis B56:

Eine Zusammensetzung aus 95.9 g Polymer STP-2, 0.4 g Vinyltriethoxysilan und 3.7 g N-(2-Aminoethyl)-3-aminopropyltriethoxysilan wurde mit verschiedenen Katalysatoren in der angegebenen Menge gemäss Tabelle 3 vermengt und die Mischung wie für Zusammensetzung B1 beschrieben auf Viskosität, Hautbildungszeit (HBZ), Oberflächenbeschaffenheit und mechanische Eigenschaften geprüft. Die Ergebnisse sind in Tabelle 3 und 4 wiedergegeben. "Zus." steht für "Zusammensetzung".

**Tabelle 3:**

| **Zus.** | **Katalysator** | **Menge** | **Konzentration ¹** | **Viskosität [Pa·s]** | | | **HBZ** | |
|---|---|---|---|---|---|---|---|---|
| | | | | **frisch** | **gelagert²** | **Zunahme** | **frisch** | **gelagert²** |
| **B32** | K-1 | 1.56 g | 3.8 | 46.2 | 50.4 | 9% | 50' | 50' |
| **B33** | K-2 | 1.41 g | 3.8 | 48.0 | 53.8 | 12% | 55' | 39' |
| **B34** | K-1 | 0.62 g | 1.5 | 40.3 | 48.0 | 19% | 72' | 70' |
| | IBAY | 0.82 g | 1.9 | | | | | |
| **B35** | K-2 | 0.55 g | 1.5 | 39.5 | 48.9 | 24% | 70' | 50' |
| | IBAY | 0.82 g | 1.9 | | | | | |
| **B36** | K-3 | 1.56 g | 3.8 | 30.4 | 33.6 | 10% | 5h 30' | 2h 12' |
| **B37** | K-4 | 1.72 g | 3.8 | 31.5 | 34.6 | 10% | 2h 7' | 4h 56' |
| **B38** | K-5 | 1.15 g | 3.8 | 35.1 | 38.5 | 10% | 60' | 80' |
| **B39** | K-5 | 0.58 g | 1.9 | 50.1 | 54.5 | 9% | 100' | 85' |
| | IBAY | 0.79 g | 1.6 | | | | | |
| **B40** | K-6 | 0.83 g | 3.8 | 33.8 | 37.3 | 10% | 65' | 60' |
| **B41** | K-8 | 1.22 g | 3.8 | 33.6 | 35.4 | 5% | 128' | 100' |
| **B42** | K-9 | 1.14 g | 3.8 | 34.6 | 35.7 | 3% | 90' | 70' |
| **B43** | K-9 | 0.58 g | 1.9 | 49.8 | 59.9 | 20% | 95' | 44' |
| | IBAY | 0.79 g | 1.6 | | | | | |
| **B44** | K-13 | 1.33 g | 3.8 | 33.9 | 42.7 | 26% | 92' | 40' |
| **B45** | K-14 | 1.18 g | 3.8 | 33.3 | 41.3 | 24% | 81' | 35' |
| **B46** | K-15 | 1.12 g | 3.8 | 34.5 | 37.2 | 8% | 56' | 36' |
| **B47** | K-15 | 0.58 g | 1.9 | 51.2 | 59.0 | 15% | 50' | 50' |
| | IBAY | 0.79 g | 1.6 | | | | | |
| **B48** | K-16 | 2.94 g | 3.8 | 32.9 | 35.4 | 8% | 75' | 75' |
| **B49** | K-17³ | 1.14 g | 3.8 | 31.3 | 33.9 | 8% | 77' | 66' |
| **B50** (Ref) | DBU | 0.55 g | 3.8 | 48.8 | 58.1 | 19% | 127' | 155' |
| **B51** (Ref) | TMG | 0.42 g | 3.8 | 44.5 | 53.4 | 20% | >12h | >12h |
| **B52** (Ref) | DHA | 0.83 g | 3.8 | 35.5 | 55.4 | 56% | 5h 30' | 4h 15' |
| **B53** (Ref) | MTHP | 0.36 g | 3.8 | 46.5 | 54.0 | 16% | 120' | 110' |
| **B54** (Ref) | TMTHP³ | 0.36 g | 3.0 | 30.0 | 34.4 | 15% | 3h 40' | 3h |
| **B55** (Ref) | DBU | 0.22 g | 1.5 | 43.2 | 45.6 | 6% | 3h 20' | 3h |
| | IBAY | 0.82 g | 1.9 | | | | | |
| **B56** (Ref) | TMG | | 1.5 | 38.2 | 42.8 | 12% | >24h | >24h |
| | IBAY | 0.17 g | 1.9 | | | | | |

| | | | | | | | | |
|---|---|---|---|---|---|---|---|---|
| ¹mmol Amidin- oder Guanidin-Gruppen bzw. Metallatome auf 100 g Silangruppen-haltiger Polyether. ² während 7 Tagen bei 60 °C in geschlossenem Gebinde. ³ als Lösung (20.0 Gew.-%) in N-Ethylpyrrolidon. | | | | | | | | |

**Tabelle 4:**

| **Zus.** | **Oberfläche nach 24h** | **Zugfestigkeit** | **Bruchdehnung** | **E-Modul** | |
|---|---|---|---|---|---|
| | | | | **0-5%** | **0-50%** |
| **B32** | fast trocken | 0.64 MPa | 170% | 0.72 MPa | 0.48 MPa |
| **B33** | fast trocken | 0.66 MPa | 176% | 0.69 MPa | 0.48 MPa |
| **B34** | fast trocken | 0.58 MPa | 138% | 0.70 MPa | 0.48 MPa |
| **B35** | fast trocken | 0.59 MPa | 140% | 0.68 MPa | 0.49 MPa |
| **B36** | trocken | 0.62 MPa | 147% | 0.40 MPa | 0.49 MPa |
| **B37** | trocken | 0.62 MPa | 125% | 0.64 MPa | 0.57 MPa |
| **B38** | trocken | 0.54 MPa | 97% | 0.86 MPa | 0.59 MPa |
| **B39** | trocken | 0.62 MPa | 112% | 0.95 MPa | 0.65 MPa |
| **B40** | trocken | 0.66 MPa | 125% | 0.87 MPa | 0.62 MPa |
| **B41** | trocken | 0.60 MPa | 111% | 0.60 MPa | 0.58 MPa |
| **B42** | trocken | 0.63 MPa | 115% | 0.85 MPa | 0.61 MPa |
| **B43** | leicht klebrig | 0.68 MPa | 142% | 0.76 MPa | 0.60 MPa |
| **B44** | trocken | 0.63 MPa | 115% | 1.02 MPa | 0.65 MPa |
| **B45** | trocken | 0.58 MPa | 96% | 0.95 MPa | 0.65 MPa |
| **B46** | trocken | 0.62 MPa | 117% | 0.75 MPa | 0.60 MPa |
| **B47** | trocken | 0.65 MPa | 121% | 0.93 MPa | 0.64 MPa |
| **B48** | trocken | 0.63 MPa | 141% | 0.77 MPa | 0.55 MPa |
| **B49** | trocken | 0.61 MPa | 112% | 0.97 MPa | 0.65 MPa |
| **B50** (Ref) | schmierig, klebrig | 0.55 MPa | 152% | 0.48 MPa | 0.44 MPa |
| **B51** (Ref) | sehr stark klebrig | n.b. | n.b. | n.b. | n.b. |
| **B52** (Ref) | stark klebrig | 0.55 MPa | 99% | 0.92 MPa | 0.57 MPa |
| **B53** (Ref) | schmierig, klebrig | 0.53 MPa | 165% | 0.52 MPa | 0.39 MPa |
| **B54** (Ref) | schmierig, klebrig | 0.45 MPa | 80% | 0.71 MPa | 0.54 MPa |
| **B55** (Ref) | leicht schmierig und klebrig | 0.66 MPa | 145% | 0.81 MPa | 0.56 MPa |
| **B56** (Ref) | (flüssig) | n.b. | n.b. | n.b. | n.b. |

| | | | | | |
|---|---|---|---|---|---|
| n.b. = nicht bestimmt bzw. nicht messbar. | | | | | |

### Zusammensetzungen B57 bis B61 und Vergleich B62:

In einem Planetenmischer wurden 36.2 g Polymer STP-1, 60.2 g gemahlene Kreide (Omyacarb® 5 GU, von Omya), 1.2 g Thixotropierpaste hergestellt wie nachstehend beschrieben, 1.2 g Vinyltrimethoxysilan, 1.2 g 3-Aminopropyltrimethoxysilan sowie verschiedene Katalysatoren in der angegebenen Menge gemäss Tabelle 5 vermengt und die Mischung wie für Zusammensetzung B1 beschrieben auf Viskosität, Hautbildungszeit (HBZ), Oberflächenbeschaffenheit und mechanische Eigenschaften geprüft. Die Ergebnisse sind in Tabelle 5 wiedergegeben. "Zus." steht für "Zusammensetzung".
Die Thixotropierpaste wurde hergestellt, indem in einem Vakuummischer 300 g Diisodecylphthalat (Palatinol® Z, von BASF) und 48 g 4,4'-Methylendiphenyldiisocyanat (Desmodur® 44 MC L, von Bayer) vorgelegt und leicht aufgewärmt und anschliessend unter starkem Rühren 27 g n-Butylamin langsam zugetropft wurden. Die entstehende Paste wurde unter Vakuum und Kühlung eine Stunde weitergerührt.

**Tabelle 5:**

| **Zus.** | **Katalysator** | **Menge** | **Konzentration¹** | **HBZ** | **Oberfläche nach 24h** | **Zugfestigkeit** | **Bruchdehnung** | **E-Modul [MPa] 0-5%** 0-100% | |
|---|---|---|---|---|---|---|---|---|---|
| **B57** | K-2 | 0.31 g | 0.8 | 11' | trocken | 3.1 MPa | 117% | 6.2 | 3.0 |
| **B58** | K-6 | 0.18 g | 0.8 | 9' | trocken | 2.8 MPa | 136% | 6.1 | 2.5 |
| **B59** | K-13 | 0.29 g | 0.8 | 20' | trocken | 2.7 MPa | 110% | 6.3 | 2.5 |
| **B60** | K-14 | 0.26 g | 0.8 | 19' | trocken | 2.8 MPa | 120% | 6.0 | 2.6 |
| **B61** | K-15 | 0.25 g | 0.8 | 9' | trocken | 2.5 MPa | 111% | 6.2 | 3.7 |
| **B62** (Ref) | DBU | 0.12 g | 0.8 | 25' | leicht schmierig | 2.5 MPa | 103% | 6.1 | 2.8 |

| | | | | | | | | | |
|---|---|---|---|---|---|---|---|---|---|
| mmol Amidin- oder Guanidin-Gruppen auf 100 g Zusammensetzung. | | | | | | | | | |

### Zusammensetzungen B63 bis B67 und Vergleiche B68 und B69:

In einem Planetenmischer wurden 36.2 g Polymer STP-2, 60.2 g gemahlene Kreide (Omyacarb® 5 GU, von Omya), 1.2 g Thixotropierpaste, hergestellt wie für Zusammensetzung Z27 beschrieben, 1.2 g Vinyltriethoxysilan, 1.2 g 3-Aminopropyltriethoxysilan sowie verschiedene Katalysatoren in der angegebenen Menge gemäss Tabelle 6 vermengt und die Mischung wie für Zusammensetzung B1 beschrieben auf Hautbildungszeit (HBZ), Oberflächenbeschaffenheit und mechanische Eigenschaften geprüft. Die Ergebnisse sind in Tabelle 6 wiedergegeben. "Zus." steht für "Zusammensetzung".

**Tabelle 6:**

| **Zus.** | **Katalysator** | **Menge** | **Konzentration¹** | **HBZ** | **Oberfläche nach 24h** | **Zugfestigkeit** | **Bruchdehnung** | **E-Modul [MPa]** | |
|---|---|---|---|---|---|---|---|---|---|
| | | | | | | | | **0-5%** | **0-100%** |
| **B63** | K-1 | 1.11 g | 2.6 | 48' | trocken | 3.5 MPa | 124% | 5.7 | 3.1 |
| **B64** | K-1 | 0.55 g | 1.3 | 50' | trocken | 2.3 MPa | 165% | 4.7 | 2.0 |
| | IBAY | 0.59 g | 1.3 | | | | | | |
| **B65** | K-5 | 0.80 g | 2.6 | 51' | trocken | 2.7 MPa | 145% | 5.0 | 2.3 |
| **B66** | K-6 | 0.59 g | 2.6 | 52' | trocken | 2.8 MPa | 139% | 5.0 | 2.4 |
| **B67** | K-15 | 0.80 g | 2.6 | 30' | trocken | 2.5 MPa | 158% | 4.3 | 2.5 |
| **B68** (Ref) | DBU | 0.40 g | 2.6 | 83' | schmierig | 2.5 MPa | 155% | 4.0 | 2.0 |
| **B69** (Ref) | DBU | 0.20 g | 1.3 | 63' | leicht | 2.2 MPa | 184% | 3.6 | 1.8 |
| | IBAY | 0.59 g | 1.3 | | schmierig | | | | |

| | | | | | | | | | |
|---|---|---|---|---|---|---|---|---|---|
| ¹ mmol Amidin- oder Guanidin-Gruppen bzw. Metallatome auf 100 g Silangruppen-haltiger Polyether. | | | | | | | | | |

### Zusammensetzung B70 (in situ-Herstellung des Katalysators):

Eine Zusammensetzung aus 20.0 g Polymer STP-1 und 0.6 g 3-Aminopropyltrimethoxysilan wurde unter Feuchtigkeitsausschluss mit 0.95 g einer Lösung von N,N'-Dicyclohexylcarbodiimid (49.8 Gew.-% in N-Ethylpyrrolidon) vermengt, die Mischung in eine innenlackierte Aluminiumtube abgefüllt und im Ofen auf 80 °C erwärmt. Nach den in der Tabelle 7 angegebenen Zeitabständen wurde die Mischung auf Hautbildungszeit (HBZ) im Normklima sowie auf den Umsatz des Carbodiimids (via Abnahme der Intensität der Carbodiimid-Bande bei ca. 2120 cm⁻¹ im FT-IR, Intensität zu Beginn = 0% Umsatz, keine Bande mehr nachweisbar = 100% Umsatz) geprüft. Die Ergebnisse sind in Tabelle 7 wiedergegeben.

**Tabelle 7:**

| **Zeit** | **HBZ** | **Carbodiimid-Umsatz** |
|---|---|---|
| 0h | 115' | 0% |
| 1h | 70' | 3% |
| 2h | 40' | 14% |
| 4h | 17' | 36% |
| 5h | 13' | 48% |
| 23h | 7' | 92% |

### Zusammensetzungen B71 bis B76 und Vergleiche B77 bis B79:

Eine Zusammensetzung aus 98 g Silangruppen-haltiger Polyether MS Polymer™ S203H (von Kaneka) und 2 g 3-Aminopropyltrimethoxysilan wurde mit verschiedenen Katalysatoren in der angegebenen Menge gemäss Tabelle 8 vermengt und die Mischung wie beschrieben auf Hautbildungszeit (HBZ) und Oberflächenbeschaffenheit nach 7 Tagen im Normklima geprüft. Die Ergebnisse sind in Tabelle 8 wiedergegeben. "Zus." steht für "Zusammensetzung".

**Tabelle 8:**

| **Zus.** | **Katalysator** | **Menge** | **Konzentration¹** | **HBZ** | **Oberfläche nach 7d** |
|---|---|---|---|---|---|
| **B71** | K-1 | 10.26 g | 24.0 | 3h | fast trocken |
| **B72** | DBTDL | 1.52 g | 2.4 | 50' | trocken |
| | K-1 | 1.71 g | 4.0 | | |
| **B73** | K-5 | 7.40 g | 24.0 | 4h 15' | fast trocken |
| **B74** | DBTDL | 0.76 g | 1.2 | 63' | trocken |
| | K-5 | 3.71 g | 12.0 | | |
| **B75** | K-15 | 7.40 g | 24.0 | 2h 25' | fast trocken |
| **B76** | DBTDL | 0.76 g | 1.2 | 75' | trocken |
| | K-15 | 3.70 g | 12.0 | | |
| **B77** (Ref) | DBU | 3.65 g | 24.0 | >12h | schmierig und klebrig |
| **B78** (Ref) | DBTDL | 1.52 g | 2.4 | 71' | trocken |
| **B79** (Ref) | DBTDL | 0.76 g | 1.2 | 85' | schmierig und klebrig |
| | DBU | 1.83 g | 12.0 | | |

| | | | | | |
|---|---|---|---|---|---|
| ¹ mmol Amidin- oder Guanidin-Gruppen bzw. Metallatome auf 100 g Silangruppen-haltiger Polyether. | | | | | |

### Zusammensetzungen B80 bis B85 und Vergleiche B86 bis B89:

Eine Zusammensetzung aus aus 96.5 g Silangruppen-haltiger Polyether TEGOPAC® Bond 150 (von Evonik), 0.5 g Vinyltriethoxysilan und 3.0 g 3-Aminopropyltriethoxysilan wurde mit verschiedenen Katalysatoren in der angegebenen Menge gemäss Tabelle 9 vermengt und die Mischung wie beschrieben auf Hautbildungszeit (HBZ) und Oberflächenbeschaffenheit nach 7 Tagen im Normklima geprüft. Die Ergebnisse sind in Tabelle 9 wiedergegeben. "Zus." steht für "Zusammensetzung".

**Tabelle 9:**

| **Zus.** | **Katalysator** | **Menge** | **Konzentration¹** | **HBZ** | **Oberfläche nach 7d** |
|---|---|---|---|---|---|
| **B80** | K-1 | 6.19 g | 15 | 56' | fast trocken |
| **B81** | K-1 | 2.15 g | 5 | 3h | fast trocken |
| | IBAY | 2.40 g | 5 | | |
| **B82** | K-5 | 4.61 g | 15 | 81' | fast trocken |
| **B83** | K5 | 1.54 g | 5 | 105' | trocken |
| | IBAY | 2.42 g | 5 | | |
| **B84** | K-15 | 4.64 g | 15 | 53' | trocken |
| **B85** | K-15 | 1.54 g | 5 | 63' | trocken |
| | IBAY | 2.42 g | 5 | | |
| **B86** (Ref) | DBU | 2.28 g | 15 | 4h 20' | schmierig |
| **B87** (Ref) | DBTDL | 0.79 g | 1.25 | 1h 45' | trocken |
| **B88** (Ref) | IBAY | 7.25 g | 15 | 2h | weich und klebrig |
| **B89** (Ref) | DBU | 4.93 g | 5 | 3h | klebrig |
| | IBAY | 2.40 g | 5 | | |

| | | | | | |
|---|---|---|---|---|---|
| mmol Amidin- oder Guanidin-Gruppen bzw. Metallatome auf 100 g Silangruppen-haltiger Polyether. | | | | | |

### Zusammensetzungen B90 bis B95 und Vergleiche B96 und B97:

Eine Zusammensetzung aus 96.0 g Silangruppen-haltiger Polyether, entweder GENIOSIL® STP-E 15 (von Wacker) oder Desmoseal® S XP 2821 (von Bayer), 0.35 g Vinyltrimethoxysilan und 3.72 g 3-Aminopropyltrimethoxysilan wurde mit verschiedenen Katalysatoren in der angegebenen Menge gemäss Tabelle 10 vermengt und die Mischung wie für Zusammensetzung B1 beschrieben geprüft. Die Ergebnisse sind in Tabelle 10 wiedergegeben. "Zus." steht für "Zusammensetzung".

**Tabelle 10:**

| **Zus.** | **Polymer** | **Katalysator, Menge** | **Konzentration¹** | **HBZ** | **Oberfläche nach 24h** | **Zugfestigkeit** | **Bruchdehnung** | **E-Modul 0-5%** |
|---|---|---|---|---|---|---|---|---|
| **B90** | E-15² | K-1, 0.81 g | 1.9 | 18' | trocken | 0.76 MPa | 48% | 1.91 MPa |
| **B91** | E-15² | K-5 0.58 | 1.9 | 15' | trocken | 0.72 MPa | 44% | 2.04 MPa |
| **B92** | E-15² | K-15 0.58 | 1.9 | 10' | trocken | 0.85 MPa | 59% | 1.94 MPa |
| **B93** | 2821³ | K-1, 0.81 g | 1.9 | 50' | trocken | 0.96 MPa | 31% | 3.3 MPa |
| **B94** | 2821³ | K-5 0.58 | 1.9 | 42' | trocken | 0.82 MPa | 28% | 3.22 MPa |
| **B95** | 2821³ | K-15 0.58 | 1.9 | 32' | trocken | 0.93 MPa | 33% | 3.21 MPa |
| **B96** (Ref) | E-15² | DBU, 0.28 g | 1.9 | 60' | schmierig | 0.72 MPa | 48% | 1.84 MPa |
| **B97** (Ref) | 2821³ | DBU, 0.28 g | 1.9 | 55' | trocken | 0.85 MPa | 28% | 3.20 MPa |

| | | | | | | | | |
|---|---|---|---|---|---|---|---|---|
| ¹ mmol Amidin- oder Guanidin-Gruppen auf 100 g Silangruppen-haltiger Polyether. ² GENIOSIL® STP-E 15 (von Wacker) ³ Desmoseal® S XP 2821 (von Bayer) | | | | | | | | |

## Patentansprüche

1. Zusammensetzung umfassend
- mindestens ein Silangruppen-haltiges organisches Polymer; und
- mindestens einen Katalysator der Formel (I), 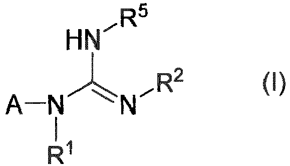 wobei
A für einen aliphatischen oder cycloaliphatischen oder arylaliphatischen Kohlenwasserstoff-Rest mit 3 bis 30 C-Atomen, welcher gegebenenfalls ungesättigte Anteile enthält, gegebenenfalls Sauerstoff- oder Stickstoffatome enthält und gegebenenfalls Aminogruppen oder Hydroxylgruppen aufweist, oder zusammen mit R¹ für einen zweiwertigen, gegebenenfalls verzweigten, Alkylen-Rest mit 4 bis 12 C-Atomen, welcher gegebenenfalls ein Heteroatom enthält, steht,
R¹ für Wasserstoff oder für einen Alkyl- oder Cycloalkyl- oder AralkylRest mit 1 bis 8 C-Atomen, oder zusammen mit A für einen zweiwertigen, gegebenenfalls verzweigten Alkylen-Rest mit 4 bis 12 C-Atomen, welcher gegebenenfalls ein Heteroatom enthält, steht, und
R² und R⁵ unabhängig voneinander jeweils für Ethyl, Isopropyl, tert.Butyl, 3-(Dimethylamino)propyl oder Cyclohexyl stehen, und
A nicht für den gleichen Rest wie R² und R⁵ steht;
wobei der Katalysator der Formel (I) kein Stickstoffatom enthält, welches direkt an einen aromatischen Ring gebunden oder Teil eines heteroaromatischen Ringsystems ist.

2. Zusammensetzung nach einem der vorhergehenden Ansprüche, **dadurch gekennzeichnet, dass** A ausgewählt ist aus der Gruppe bestehend aus n-Hexyl, n-Octyl, 2-Ethylhexyl, n-Decyl, Lauryl, Cyclohexyl, Benzyl, 2-Methoxyethyl, 3-Methoxypropyl, Polyoxyalkylen-Rest mit Oxyethylen- und 1,2-Oxypropyleneinheiten und einem Molekulargewicht im Bereich von 180 bis 600 g/mol, N-Methyl-3-aminopropyl, N-(2-Ethylhexyl)-3-aminopropyl, N-Cyclohexyl-3-aminopropyl, 3-(N,N-Dimethylamino)propyl, 2-Aminopropyl, 3-Aminopropyl, 3-Aminopentyl, 5-Amino-4-methylpentyl, 5-Amino-2-methylpentyl, 6-Aminohexyl, 6-Amino-3,3(5),5-trimethylhexyl, 6-Amino-2,2(4),4-trimethylhexyl, 8-Aminooctyl, 10-Aminodecyl, 12-Aminododecyl, 3-Aminomethyl-3,5,5-trimethylcyclohexyl, 3-Amino-1,5,5-trime-thylcyclohexylmethyl, 3-Aminomethyl-cyclohexylmethyl, 4-Aminomethyl-cyclohexylmethyl, 3-Aminomethyl-benzyl, 5-Amino-3-oxa-pentyl, ω-2-Aminopropyl-polyoxypropylen-Rest mit einem Molekulargewicht im Bereich von etwa 200 bis 500 g/mol, 2-Hydroxypropyl, 3-Hydroxypropyl, 1,1-Dimethyl-2-hydroxyethyl und 5-Hydroxy-3-oxa-pentyl.

3. Zusammensetzung nach einem der vorhergehenden Ansprüche, **dadurch gekennzeichnet, dass** R¹ für Wasserstoff steht.

4. Zusammensetzung nach einem der vorhergehenden Ansprüche, **dadurch gekennzeichnet, dass** das Silangruppen-haltige organische Polymer ein Silangruppen-haltiges Polyolefin oder ein Silangruppen-haltiger Polyester oder ein Silangruppen-haltiges Poly(meth)acrylat oder ein Silangruppen-haltiger Polyether oder eine Mischform dieser Polymere ist.

5. Zusammensetzung nach einem der vorhergehenden Ansprüche, **dadurch gekennzeichnet, dass** zusätzlich mindestens ein Organotitanat enthalten ist.

6. Verfahren zur Herstellung einer Zusammensetzung nach einem der Ansprüche 1 bis 5, umfassend einen Schritt zur Herstellung eines Katalysators der Formel (I), **dadurch gekennzeichnet, dass** mindestens ein Amin der Formel (II) mit mindestens einem Carbodiimid der Formel (III) umgesetzt wird. 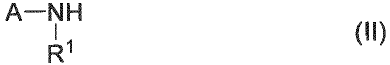
R⁵-N=C=N-R² (III)

7. Verfahren nach Anspruch 6, **dadurch gekennzeichnet, dass** das Amin der Formel (II) ausgewählt ist aus der Gruppe bestehend aus n-Hexylamin, n-Octylamin, 2-Ethylhexylamin, n-Decylamin, Laurylamin, Cyclohexylamin, Benzylamin, 2-Methoxyethylamin, 3-Methoxypropylamin, Polyoxyalkylenamin mit Oxyethylen- und 1,2-Oxypropyleneinheiten und einem mittleren Molekulargeicht im Bereich von 180 bis 600 g/mol, N-Methyl-1,3-propandiamin, N-(2-Ethylhexyl)-1,3-propandiamin, N-Cyclohexyl-1,3-propandiamin, N,N-Dimethyl-1,3-propandiamin, 1,2-Propandiamin, 1,3-Propandiamin, 1,3-Pentandiamin, 1,5-Diamino-2-methylpentan, 1,6-Hexandiamin, 2,2,4- und 2,4,4-Trimethylhexamethylendiamin, 1,8-Octandiamin, 1,10-Decandiamin, 1,12-Dodecandiamin, 1-Amino-3-aminomethyl-3,5,5-trimethylcyclohexan, 1,3-Bis(aminomethyl)cyclohexan, 1,4-Bis(aminomethyl)cyclohexan,1,3-Bis(aminomethyl)benzol, Bis-(2-aminoethyl)ether, Polyoxypropylendiamin mit einem mittleren Molekulargewicht im Bereich von etwa 220 bis 500 g/mol, 1-Amino-2-propanol, 3-Amino-1-propanol, 2-Amino-2-methyl-1-propanol und 2-(2-Aminoethoxy)ethanol.

8. Verfahren nach einem der Ansprüche 6 oder 7, **dadurch gekennzeichnet, dass** das Carbodiimid der Formel (III) ausgewählt ist aus der Gruppe bestehend aus N,N'-Diisopropylcarbodiimid, N,N'-Di-tert.butylcarbodiimid, N,N'-Dicyclohexylcarbodiimid und N-Ethyl-N'-(3-dimethylaminopropyl)carbodiimid.

9. Verfahren nach einem der Ansprüche 6 bis 8, **dadurch gekennzeichnet, dass** die Umsetzung in Gegenwart des Silangruppen-haltigen organischen Polymers erfolgt.

10. Verwendung einer Zusammensetzung nach einem der Ansprüche 1 bis 5 als Klebstoff, Dichtstoff oder Beschichtung.

11. Ausgehärtete Zusammensetzung, erhalten aus einer Zusammensetzung nach einem der Ansprüche 1 bis 5, nach deren Härtung mit Wasser, insbesondere in Form von Luftfeuchtigkeit.

12. Verwendung eines Katalysators der Formel (I), wie er in Anspruch 1 beschrieben worden ist, als Vernetzungskatalysator für härtbare Zusammensetzungen.

13. Katalysator der Formel (Ia), 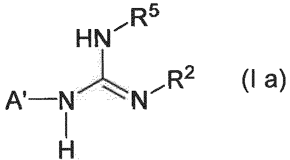 wobei
A' für einen aliphatischen oder cycloaliphatischen oder arylaliphatischen Kohlenwasserstoff-Rest mit 6 bis 30 C-Atomen, welcher gegebenenfalls Ethersauerstoffe enthält, und welcher keine Silangruppen, keine Hydroxylgruppen und keine Aminogruppen aufweist, steht, und
R² und R⁵ unabhängig voneinander jeweils für Ethyl, Isopropyl, tert.Butyl, 3-(Dimethylamino)propyl oder Cyclohexyl stehen, und
A' nicht für den gleichen Rest wie R² und R⁵ steht.

14. Katalysator der Formel (Ib), 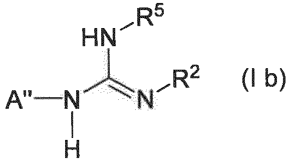 wobei
A" für einen aliphatischen oder cycloaliphatischen oder arylaliphatischen Kohlenwasserstoff-Rest mit 3 bis 30 C-Atomen, welcher frei ist von Silangruppen, gegebenenfalls Ethersauerstoffe enthält und mindestens eine Hydroxylgruppe oder primäre oder sekundäre Aminogruppe aufweist, steht, und
R² und R⁵ unabhängig voneinander jeweils für Ethyl, Isopropyl, tert.Butyl, 3-(Dimethylamino)propyl oder Cyclohexyl stehen, und
A" nicht für den gleichen Rest wie R² und R⁵ steht.

15. Katalysator der Formel (Ic), 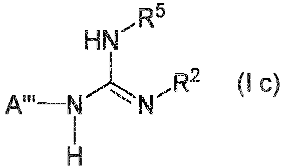 wobei
A'" für einen aliphatischen oder cycloaliphatischen oder arylaliphatischen Kohlenwasserstoff-Rest mit 3 bis 30 C-Atomen, welcher frei ist von Silangruppen, gegebenenfalls Ethersauerstoff enthält und mindestens eine tertiäre Aminogruppe aufweist, steht, und
R² und R⁵ unabhängig voneinander jeweils für Ethyl, Isopropyl, tert.Butyl, 3-(Dimethylamino)propyl oder Cyclohexyl stehen, und
A'" nicht für den gleichen Rest wie R² und R⁵ steht.

## Claims

1. Composition comprising
- at least one organic polymer containing silane groups; and
- at least one catalyst of the formula (I), 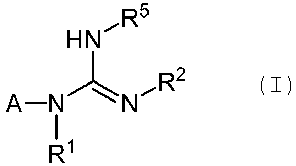 where
A is an aliphatic or cycloaliphatic or arylaliphatic hydrocarbyl radical which has 3 to 30 carbon atoms, optionally contains unsaturated moieties, optionally contains oxygen or nitrogen atoms and optionally has amino groups or hydroxyl groups, or together with R¹ is a divalent, optionally branched alkylene radical which has 4 to 12 carbon atoms and optionally contains a heteroatom,
R¹ is hydrogen or an alkyl or cycloalkyl or aralkyl radical having 1 to 8 carbon atoms, or together with A is a divalent, optionally branched alkylene radical which has 4 to 12 carbon atoms and optionally contains a heteroatom, and
R² and R⁵ are each independently ethyl, isopropyl, tert-butyl, 3-(dimethylamino)propyl or cyclohexyl, and
A is not the same radical as R² and R⁵;
where the catalyst of the formula (I) does not contain any nitrogen atom bonded directly to an aromatic ring or part of a heteroaromatic ring system.

2. Composition according to any of the preceding claims, **characterized in that** A is selected from the group consisting of n-hexyl, n-octyl, 2-ethylhexyl, n-decyl, lauryl, cyclohexyl, benzyl, 2-methoxyethyl, 3-methoxypropyl, polyoxyalkylene radical having oxyethylene and 1,2-oxypropylene units and a molecular weight in the range from 180 to 600 g/mol, N-methyl-3-aminopropyl, N-(2-ethyl-hexyl)-3-aminopropyl, N-cyclohexyl-3-aminopropyl, 3-(N,N-dimethylamino)propyl, 2-aminopropyl, 3-aminopropyl, 3-aminopentyl, 5-amino-4-methylpentyl, 5-amino-2-methylpentyl, 6-aminohexyl, 6-amino-3,3(5),5-trimethylhexyl, 6-amino-2,2(4),4-trimethylhexyl, 8-aminooctyl, 10-aminodecyl, 12-aminododecyl, 3-aminomethyl-3,5,5-trimethylcyclo-hexyl, 3-amino-1,5,5-trimethylcyclo-hexylmethyl, 3-aminomethylcyclohexylmethyl, 4-aminomethylcyclo-hexylmethyl, 3-aminomethylbenzyl, 5-amino-3-oxapentyl, ω-2-aminopropylpolyoxypropylene radical having a molecular weight in the range from about 200 to 500 g/mol, 2-hydroxypropyl, 3-hydroxypropyl, 1,1-dimethyl-2-hydroxyethyl and 5-hydroxy-3-oxapentyl.

3. Composition according to either of the preceding claims, **characterized in that** R¹ is hydrogen.

4. Composition according to any of the preceding claims, **characterized in that** the organic polymer containing silane groups is a polyolefin containing silane groups or a polyester containing silane groups or a poly(meth)acrylate containing silane groups or a polyether containing silane groups or a mixed form of these polymers.

5. Composition according to any of the preceding claims, **characterized in that** at least one organotitanate is additionally present.

6. Process for producing a composition according to any of Claims 1 to 5, comprising a step for preparing a catalyst of the formula (I), **characterized in that** at least one amine of the formula (II) is reacted with at least one carbodiimide of the formula (III). 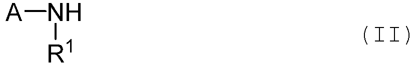
**R⁵-N=C=N-R²** (III)

7. Process according to Claim 6, **characterized in that** the amine of the formula (II) is selected from the group consisting of n-hexylamine, n-octylamine, 2-ethylhexylamine, n-decylamine, laurylamine, cyclohexylamine, benzylamine, 2-methoxyethylamine, 3-methoxypropylamine, polyoxyalkyleneamine having oxyethylene and 1,2-oxypropylene units and a mean molecular weight in the range from 180 to 600 g/mol, N-methyl-1,3-propanediamine, N-(2-ethylhexyl)-1,3-propanediamine, N-cyclohexyl-1,3-propanediamine, N,N-dimethyl-1,3-propanediamine, 1,2-propanediamine, 1,3-propanediamine, 1,3-pentanediamine, 1,5-diamino-2-methylpentane, 1,6-hexanediamine, 2,2,4- and 2,4,4-trimethylhexa-methylenediamine, 1,8-octanediamine, 1,10-decanediamine, 1,12-dodecanediamine, 1-amino-3-aminomethyl-3,5,5-trimethylcyclohexane, 1,3-bis(aminomethyl)cyclohexane, 1,4-bis(aminomethyl)-cyclohexane, 1,3-bis(aminomethyl)benzene, bis(2-aminoethyl) ether, polyoxypropylenediamine having a mean molecular weight in the range from about 220 to 500 g/mol, 1-amino-2-propanol, 3-amino-1-propanol, 2-amino-2-methyl-1-propanol and 2-(2-aminoethoxy)ethanol.

8. Process according to either of Claims 6 and 7, **characterized in that** the carbodiimide of the formula (III) is selected from the group consisting of N,N'-diisopropylcarbodiimide, N,N'-di-tert-butylcarbodiimide, N,N'-dicyclohexylcarbodiimide and N-ethyl-N'-(3-dimethylaminopropyl)-carbodiimide.

9. Process according to any of Claims 6 to 8, **characterized in that** the reaction is effected in the presence of the organic polymer containing silane groups.

10. Use of a composition according to any of Claims 1 to 5 as adhesive, sealant or coating.

11. Cured composition obtained from a composition according to any of Claims 1 to 5 after it has been cured with water, especially in the form of air humidity.

12. Use of a catalyst of the formula (I) as described in Claim 1 as crosslinking catalyst for curable compositions.

13. Catalyst of the formula (I a) 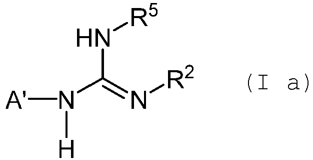 where
A' is an aliphatic or cycloaliphatic or arylaliphatic hydrocarbyl radical which has 6 to 30 carbon atoms and optionally contains ether oxygens, and which does not have any silane groups, any hydroxyl groups or any amino groups, and
R² and R⁵ are each independently ethyl, isopropyl, tert-butyl, 3-(dimethylamino)propyl or cyclohexyl, and
A' is not the same radical as R² and R⁵.

14. Catalyst of the formula (I b) 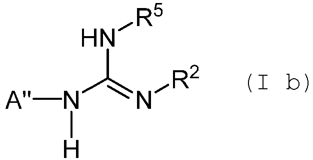 where
A" is an aliphatic or cycloaliphatic or arylaliphatic hydrocarbyl radical which has 3 to 30 carbon atoms and is free of silane groups, optionally contains ether oxygens and has at least one hydroxyl group or primary or secondary amino group, and
R² and R⁵ are each independently ethyl, isopropyl, tert-butyl, 3-(dimethylamino)propyl or cyclohexyl, and
A" is not the same radical as R² and R⁵.

15. Catalyst of the formula (I c) 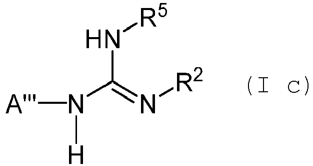 where
A"' is an aliphatic or cycloaliphatic or arylaliphatic hydrocarbyl radical which has 3 to 30 carbon atoms, is free of silane groups, optionally contains ether oxygen and has at least one tertiary amino group, and
R² and R⁵ are each independently ethyl, isopropyl, tert-butyl, 3-(dimethylamino)propyl or cyclohexyl, and
A"' is not the same radical as R² and R⁵.

## Revendications

1. Composition comprenant
- au moins un polymère organique contenant des groupes silane ; et
- au moins un catalyseur de formule (I), 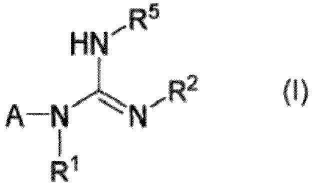
A représentant un radical hydrocarboné aliphatique ou cycloaliphatique ou arylaliphatique comportant 3 à 30 atomes de C, qui contient éventuellement des portions insaturées, qui contient éventuellement des atomes d'oxygène ou des atomes d'azote et qui présente éventuellement des groupes amino ou des groupes hydroxyle, ou conjointement avec R¹, représentant un radical alkylène divalent, éventuellement ramifié, comportant 4 à 12 atomes de C, qui contient éventuellement un hétéroatome,
R¹ représentant hydrogène ou un radical alkyle ou cycloalkyle ou aralkyle comportant 1 à 8 atomes de C, ou conjointement avec A représentant un radical alkylène divalent, éventuellement ramifié, comportant 4 à 12 atomes de C, qui contient éventuellement un hétéroatome, et
R² et R⁵ représentant indépendamment l'un de l'autre à chaque fois éthyle, isopropyle, tert-butyle, 3-(diméthylamino)propyle ou cyclohexyle, et
A ne représentant pas le même radical que R² et R⁵ ;
le catalyseur de formule (I) ne contenant pas d'atome d'azote qui est directement lié à un cycle aromatique ou qui fait partie d'un système cyclique hétéroaromatique.

2. Composition selon l'une quelconque des revendications précédentes, **caractérisée en ce que** A est choisi dans le groupe constitué par n-hexyle, n-octyle, 2-éthylhexyle, n-décyle, lauryle, cyclohexyle, benzyle, 2-méthoxyéthyle, 3-méthoxypropyle, un radical polyoxyalkylène comportant des motifs oxyéthylène et 1,2-oxypropylène et un poids moléculaire dans la plage de 180 à 600 g/mole, N-méthyl-3-aminopropyle, N-(2-éthylhexyl)-3-aminopropyle, N-cyclohexyl-3-aminopropyle, 3-(N,N-diméthylamino)propyle, 2-aminopropyle, 3-aminopropyle, 3-aminopentyle, 5-amino-4-méthylpentyle, 5-amino-2-méthylpentyle, 6-aminohexyle, 6-amino-3,3(5),5-triméthylhexyle, 6-amino-2,2(4),4-triméthylhexyle, 8-aminooctyle, 10-aminodécyle, 12-amino-dodécyle, 3-aminométhyl-3,5,5-triméthylcyclohexyle, 3-amino-1,5,5-triméthylcyclohexylméthyle, 3-aminométhyl-cyclohexylméthyle, 4-aminométhyl-cyclohexylméthyle, 3-aminométhyl-benzyle, 5-amino-3-oxa-pentyle, radical ω-2-aminopropyl-polyoxypropylène doté d'un poids moléculaire dans la plage d'environ 200 à 500 g/mole, 2-hydroxypropyle, 3-hydroxypropyle, 1,1-diméthyl-2-hydroxyéthyle et 5-hydroxy-3-oxa-pentyle.

3. Composition selon l'une quelconque des revendications précédentes, **caractérisée en ce que** R¹ représente hydrogène.

4. Composition selon l'une quelconque des revendications précédentes, **caractérisée en ce que** le polymère organique contenant des groupes silane est une polyoléfine contenant des groupes silane ou un polyester contenant des groupes silane ou un poly(méth)acrylate contenant des groupes silane ou un polyéther contenant des groupes silane ou une forme mixte de ces polymères.

5. Composition selon l'une quelconque des revendications précédentes, **caractérisée en ce qu'**au moins un organotitanate est de plus contenu.

6. Procédé pour la préparation d'une composition selon l'une quelconque des revendications 1 à 5, comprenant une étape pour la préparation d'un catalyseur de formule (I), **caractérisé en ce qu'**au moins une amine de formule (II) est transformée avec au moins un carbodiimide de formule (III) 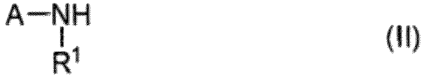
**R⁵-N=C=N-R²** (III)

7. Procédé selon la revendication 6, **caractérisé en ce que** l'amine de formule (II) est choisie dans le groupe constitué par la n-hexylamine, la n-octylamine, la 2-éthylhexylamine, la n-décylamine, la laurylamine, la cyclohexylamine, la benzylamine, la 2-méthoxyéthylamine, la 3-méthoxypropylamine, une polyoxyalkylèneamine possédant des motifs oxyéthylène et 1,2-oxypropylène et un poids moléculaire moyen dans la plage de 180 à 600 g/mole, la N-méthyl-1,3-propanediamine, la N-(2-éthylhexyl)-1,3-propanediamine, la N-cyclohexyl-1,3-propanediamine, la N,N-diméthyl-1,3-propanediamine, la 1,2-propanediamine, la 1,3-propanediamine, la 1,3-pentanediamine, le 1,5-diamino-2-méthylpentane, la 1,6-hexanediamine, la 2,2,4-triméthylhexaméthylènediamine et la 2,4,4-triméthylhexaméthylènediamine, la 1,8-octanediamine, la 1,10-décanediamine, la 1,12-dodécanediamine, le 1-amino-3-aminométhyl-3,5,5-triméthylcyclohexane, le 1,3-bis(aminométhyl)cyclohexane, le 1,4-bis(aminométhyl)cyclohexane, le 1,3-bis(aminométhyl)benzène, le bis(2-aminoéthyl)éther, une polyoxypropylènediamine possédant un poids moléculaire moyen dans la plage d'environ 220 à 500 g/mole, le 1-amino-2-propanol, le 3-amino-1-propanol, le 2-amino-2-méthyl-1-propanol et le 2-(2-aminoéthoxy)éthanol.

8. Procédé selon l'une quelconque des revendications 6 et 7, **caractérisé en ce que** le carbodiimide de formule (III) est choisi dans le groupe constitué par le N,N'-diisopropylcarbodiimide, le N,N'-di-tert-butylcarbodiimide, le N,N'-dicyclohexylcarbodiimide et le N-éthyl-N'-(3-dimethylaminopropyl)carbodiimide.

9. Procédé selon l'une quelconque des revendications 6 à 8, **caractérisé en ce que** la transformation est réalisée en présence du polymère organique contenant des groupes silane.

10. Utilisation d'une composition selon l'une quelconque des revendications 1 à 5 en tant qu'adhésif, agent d'étanchéité ou revêtement.

11. Composition durcie, obtenue à partir d'une composition selon l'une quelconque des revendications 1 à 5, après son durcissement avec de l'eau, en particulier sous forme d'humidité de l'air.

12. Utilisation d'un catalyseur de formule (I), tel qu'il a été décrit dans la revendication 1, en tant que catalyseur de réticulation pour des compositions durcissables.

13. Catalyseur de formule (I a), 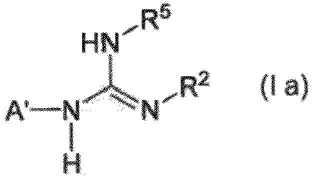
A' représentant un radical hydrocarboné aliphatique ou cycloaliphatique ou arylaliphatique comportant 6 à 30 atomes de C, qui contient éventuellement des oxygènes d'éther, et qui ne présente aucun groupe silane, aucun groupe hydroxyle et aucun groupe amino, et
R² et R⁵ représentant indépendamment l'un de l'autre à chaque fois éthyle, isopropyle, tert-butyle, 3-(diméthylamino)propyle ou cyclohexyle, et
A' ne représentant pas le même radical que R² et R⁵.

14. Catalyseur de formule (I b), 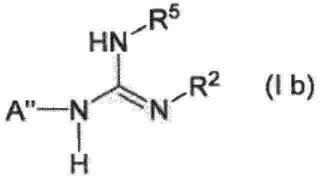
A'' représentant un radical hydrocarboné aliphatique ou cycloaliphatique ou arylaliphatique comportant 3 à 30 atomes de C, qui est exempt de groupes silane, qui contient éventuellement des oxygènes d'éther, et qui présente au moins un groupe hydroxyle ou groupe amino primaire ou secondaire, et
R² et R⁵ représentant indépendamment l'un de l'autre à chaque fois éthyle, isopropyle, tert-butyle, 3-(diméthylamino)propyle ou cyclohexyle, et
A" ne représentant pas le même radical que R² et R⁵.

15. Catalyseur de formule (I c), 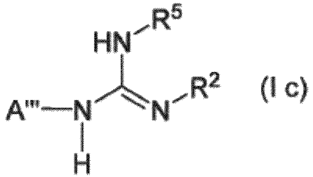
A"' représentant un radical hydrocarboné aliphatique ou cycloaliphatique ou arylaliphatique comportant 3 à 30 atomes de C, qui est exempt de groupes silane, qui contient éventuellement un oxygène d'éther et qui présente au moins un groupe amino tertiaire, et
R² et R⁵ représentant indépendamment l'un de l'autre à chaque fois éthyle, isopropyle, tert-butyle, 3-(diméthylamino)propyle ou cyclohexyle, et
A"' ne représentant pas le même radical que R² et R⁵.
